# EUROPEAN PATENT APPLICATION

(11) **EP 4 295 862 A2**
(43) Date of publication of application: **27.12.2023**
(21) Application number: 23181557.2
(22) Date of filing: 26.06.2023
(51) Int. Cl.: A61K 39/12, A61P 31/14

(54) **CORONAVIRUS VACCINE**

(30) Priority: 26.06.2022 US 202263355648 P; 30.06.2022 US 202263357628 P; 05.07.2022 US 202263358522 P; 02.08.2022 US 202263394571 P; 30.08.2022 US 202263402444 P; 19.10.2022 US 202263417680 P; 03.11.2022 US 202263422404 P; 14.11.2022 US 202263425290 P; 29.11.2022 US 202218071499; 24.02.2023 US 202363486953 P; 14.03.2023 US 202363452148 P; 10.05.2023 US 202363465521 P; 29.05.2023 US 202363469472 P
(71) Applicant: BioNTech SE, 55131 Mainz (DE)
(72) Inventor: MUIK, Alexander, 55131 Mainz (DE); PORAN, Asaf, Cambridge, 02139 (US); SAHIN, Ugur, 55131 Mainz (DE); SWANSON, Kena Anne, Pearl River, 10965 (US); YANG, Qi, Pearl River, 10965 (US); CAI, Hui, Pearl River, 10965 (US); MODJARRAD, Kayvon, Pearl River, NY, 10965 (US)
(74) Representative: Thomann, William John

(57) **Abstract**

This disclosure relates to the field of RNA to prevent or treat coronavirus infection. In particular, the present disclosure relates to methods and agents for vaccination against coronavirus infection and inducing effective coronavirus antigen-specific immune responses such as antibody and/or T cell responses.

## Description

This disclosure relates to the field of RNA to prevent or treat coronavirus infection. In particular, the present disclosure relates to methods and agents for vaccination against coronavirus infection and inducing effective coronavirus antigen-specific immune responses such as antibody and/or T cell responses. These methods and agents are, in particular, useful for the prevention or treatment of coronavirus infection. Administration of RNA disclosed herein to a subject can protect the subject against coronavirus infection. Specifically, in one embodiment, the present disclosure relates to methods comprising administering to a subject RNA encoding a peptide or protein comprising an epitope of SARS-CoV-2 spike protein (S protein) for inducing an immune response against coronavirus S protein, in particular S protein of SARS-CoV-2, in the subject, i.e., vaccine RNA encoding vaccine antigen. Administering to the subject RNA encoding vaccine antigen may provide (following expression of the RNA by appropriate target cells) vaccine antigen for inducing an immune response against vaccine antigen (and disease-associated antigen) in the subject.

SARS-CoV-2 infections and the resulting disease COVID-19 have spread globally, affecting a growing number of countries. On 11 March 2020 the WHO characterized the COVID-19 outbreak as a pandemic. As of 01 December 2020, there have been >63 million globally confirmed COVID-19 cases and >1.4 million deaths, with 191 countries/regions affected. The ongoing pandemic remains a significant challenge to public health and economic stability worldwide.

The present invention is directed to a composition comprising an RNA molecule having:
(a) a nucleotide sequence that is at least 99% identical to SEQ ID NO: 161;
(b) a nucleotide sequence that is at least 95% identical to SEQ ID NO: 161, and which encodes a SARS-CoV-2 S protein comprising the following mutations relative to SEQ ID NO: 1: T19I, Δ24-26, A27S, V83A, G142D, Δ145, H146Q, Q183E, V213E, G252V, G339H, R346T, L368I, S371F, S373P, S375F, T376A, D405N, R408S, K417N, N440K, V445P, G446S, N460K, S477N, T478K, E484A, F486P, F490S, Q498R, N501Y, Y505H, D614G, H655Y, N679K, P681H, N764K, D796Y, Q954H, N969K, K986P, and V987P; or
(c) a nucleotide sequence as set forth in SEQ ID NO: 161; and
wherein the RNA molecule comprises:
(i) a 5' cap; and
(ii) a modified uridine in place of each uridine.

The present invention is also directed to a composition of the invention for use in a method of inducing an immune response against coronavirus in a subject, said method comprising administering to a subject the composition.

In some embodiments, the modified uridine can be N1-methyl-pseudouridine.

In some embodiments, the 5' cap can comprise m₂^{7,3'-O}Gppp(m₁^{2'-O})ApG.

In come embodiments, the RNA molecule can be encapsulated in a lipid nanoparticle (LNP), preferably wherein the LNP comprises molar ratios of 20-60% ionizable cationic lipid, 5-25% neutral lipid, 25-55% sterol, and 0.5-15% PEG-modified lipid.

In some embodiments, the composition can comprise one or more additional RNA molecules, each having a nucleotide sequence encoding an S protein of a SARS-CoV-2 strain or variant that is not XBB.1.5, preferably wherein the one or more additional RNA molecules comprise a sequence that is at least 95% identical to that set forth in SEQ ID NO: 20, 72, or 103.

In some embodiments, the RNA molecule comprises:
(i) N1-methyl-pseudouridine in place of each uridine; and
(ii) a 5' cap that comprises m₂^{7,3'-O}Gppp(m₁^{2'-O})ApG;
wherein the RNA molecule is encapsulated in a lipid nanoparticle (LNP); and
wherein the LNP comprises molar ratios of 20-60% ionizable cationic lipid, 5-25% neutral lipid, 25-55% sterol, and 0.5-15% PEG-modified lipid.

In some embodiments, the composition can comprise about 10 mM Tris buffer and about 10% sucrose.

In some embodiments, the composition can comprise at least one unit dose of LNP-encapsulated RNA molecules, optionally wherein the unit dose comprises the RNA molecule in an amount of about 30 µg, or wherein the unit dose comprises the RNA molecule in an amount of about 10 µg, or wherein the unit dose comprises the RNA molecule in an amount of about 3 µg.

In some embodiments, the composition is formulated as a multi-dose formulation in a vial.

In some embodiments, the subject can be 12 years or older, and the composition can comprise 30 µg of the RNA molecule, or the subject can be 5 years to less than 12 years old, and the composition can comprise 10 µg of the RNA molecule, or the subject can be 6 months to less than 5 years old, and the composition can comprise 3 µg of the RNA molecule.

In some embodiments, the composition can be administered in a volume of about 200 µL to 300 µL.

In some embodiments, the subject was previously administered one or more doses of a SARS-CoV-2 vaccine, preferably wherein the subject was previously administered a complete dosing regimen of a SARS-CoV-2 vaccine.

In some embodiments, the subject was previously administered a first dose and a second dose of BNT162b2, wherein the first dose and the second dose were administered about 21 days apart, and/or wherein the subject was previously administered as a booster dose a bivalent vaccine that delivers (i) a SARS-CoV-2 S protein of an Omicron BA.4/5 variant and (ii) a SARS-CoV-2 S protein of a Wuhan strain.

In some embodiments, said method can further comprise administering one or more vaccines against a non-SARS-CoV-2 disease, preferably wherein the one or more vaccines comprises an RSV vaccine, an influenza vaccine, or a combination thereof.

In some embodiments, compositions disclosed herein (e.g., monovalent compositions comprising RNA encoding a SARS-CoV-2 S protein of an XBB.1.5 variant) can induce a strong immune response (e.g., high neutralization titers) against certain SARS-CoV-2 variants of concern (e.g., XBB variants of concern (including, e.g., an XBB.1.5 variant, an XBB.1.16 variant, an XBB.2.3 variant, and/or an XBB.2.3.2 variant)). In some embodiments, such compositions are monovalent compositions comprising RNA encoding a SARS-CoV-2 S protein of an XBB.1.5 variant. In some embodiments, such compositions are monovalent compositions comprising RNA encoding a SARS-CoV-2 S protein of an XBB.1.16 variant. In some embodiments, such compositions are monovalent compositions comprising RNA encoding a SARS-CoV-2 S protein of an XBB.2.3 variant. In some embodiments, such compositions are monovalent compositions comprising RNA encoding a SARS-CoV-2 S protein of an XBB.2.3.2 variant. As demonstrated herein, in some embodiments, such compositions can induce surprisingly high neutralization titers against certain XBB variants of concern (including, e.g., XBB.1.5, XBB.1.16, and XBB.2.3 variants of concern). Even more surprisingly, in some embodiments, such compositions can induce neutralization titers that are higher against a given variant than a variant-matched vaccine (e.g., in some embodiments, RNA described herein comprising a nucleotide sequence that encodes an XBB.1.5 S protein, can induce neutralization titers against a XBB.1.16 variant of concern that are higher than those induced by an XBB.1.16-adapted vaccine). In some embodiments, the strong immune response can be observed in vaccine-naïve subjects (e.g., young pediatric patients (e.g., patients 6 months to less than 5 years old). In some embodiments, the strong immune response can be observed in subjects without a previous or current coronavirus infection (e.g., a SARS-CoV-2 infection). In some embodiments, a strong immune response can be observed in subjects who previously received a SARS-CoV-2 vaccine (e.g., in some embodiments an RNA vaccine encoding a SARS-CoV-2 S protein, e.g., in some embodiments of a Wuhan strain) and/or who were previously infected with SARS-CoV-2. In some embodiments, such broader cross-neutralization can be observed in young pediatric subjects (e.g., subjects aged 6 months to less than 2 years, and/or 2 years to less than 5 years).

### Brief Description of the Figures

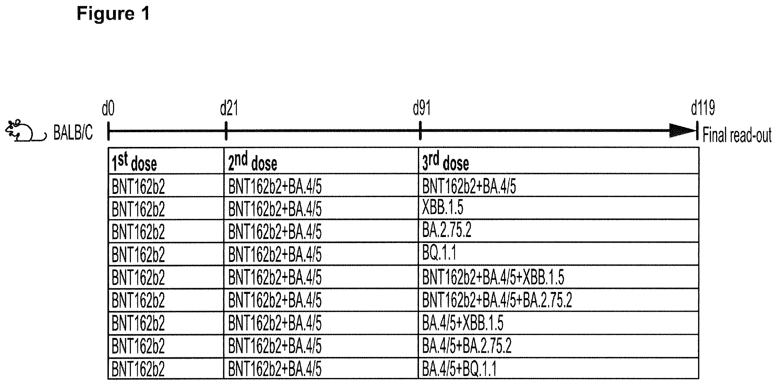

**Fig. 1****. Experimental design for a study to characterize immune responses induced by variant-adapted vaccines in SARS-CoV-2 vaccine-experienced mice.** Mice are split into 9 groups, each of which is administered a first dose of an RNA encoding a SARS-CoV-2 S protein of a Wuhan strain (BNT162b2) and a second dose of a bivalent composition comprising a first RNA encoding a SARS-CoV-2 S protein of a Wuhan strain and a second RNA encoding a SARS-COV-2 S protein of an Omicron BA.4/5 variant (BNT162b2 + BA.4/5). Following the second dose, mice are administered a composition comprising an RNA encoding a SARS-CoV-2 S protein of a strain or variant or combination of strains/variants, as indicated in the Figure. The first and second doses are administered 21 days apart, and the third dose is administered about 70 days after the third dose. 119 days after administering the first dose, mice are sacrificed and the experiment concluded.

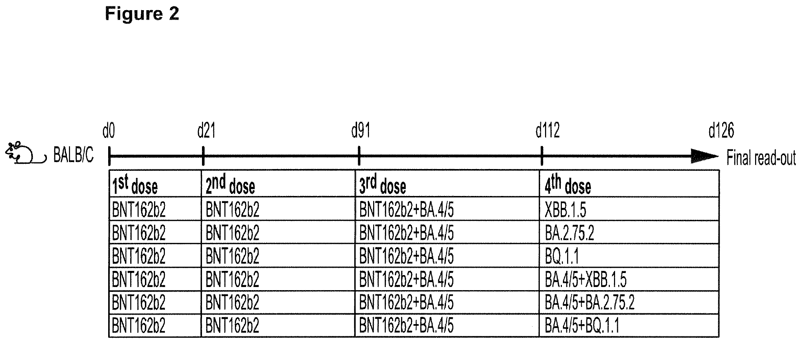

**Fig. 2****. Experimental design for a study to characterize variant-adapted vaccines in SARS-CoV-2 vaccine-experienced mice.** Mice are split into 6 groups, each of which is administered a first and a second dose of an RNA encoding a SARS-CoV-2 S protein of a Wuhan strain (BNT162b2), followed by a third dose of a bivalent composition comprising a first RNA encoding a SARS-CoV-2 S protein of a Wuhan strain and a second RNA encoding a SARS-CoV-2 S protein of a BA.4/5 Omicron variant (BNT162b2 + BA.4/5). Following the third dose, mice are administered a composition comprising an RNA encoding an SARS-CoV-2 S protein of a variant or strain or combination of strains/variants, as indicated in the Figure. The first and second doses are administered 21 days apart, the third dose is administered 70 days after the third dose, and the 4th dose is administered 21 days after the third dose. 126 days after administering the first dose, mice are sacrificed and the experiment concluded.

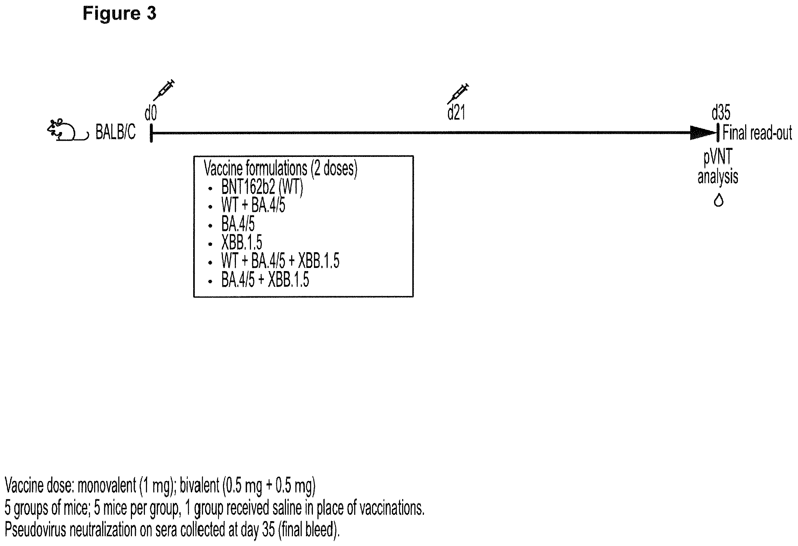

**Fig. 3****. Exemplary experimental protocol for testing variant-adapted vaccines as a primary series in mice.** Two doses of a monovalent or bivalent vaccine composition comprising mRNA(s) encoding the SARS-CoV-2 S protein(s) of the strains/variants indicated in the figure were administered to mice 21 days apart. "WT" corresponds to mRNA encoding a SARS-CoV-2 S protein from a Wuhan strain. Indicated in the figure is the mass of RNA (µg) administered to mice.

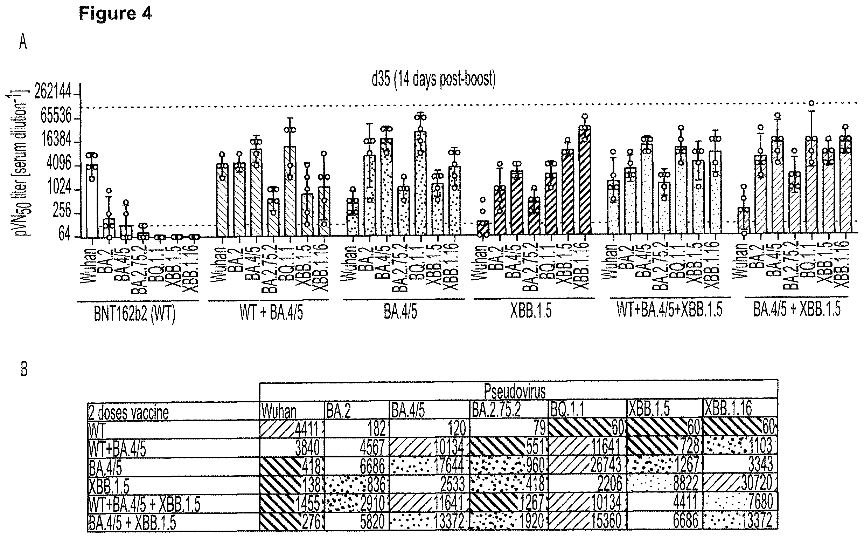

**Fig. 4****. A monovalent XBB.1.5-adapted vaccine, as a primary series, elicits higher neutralization titers against XBB.1.5 than a bivalent Wuhan** + **BA.4/5 vaccine.** Shown are results collected in performing the experiment summarized in Figure 3. (A) shows pseudovirus neutralization titers of serum samples collected at Day 35 of the experiment summarized in Figure 3. (B) Lists neutralization titer values for the bar graph shown in panel (A).

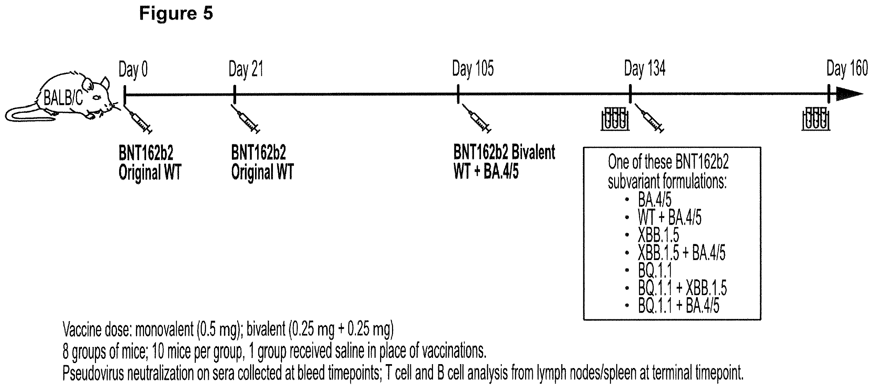

**Fig. 5****. Exemplary experimental protocol for testing variant-adapted vaccines as a fourth booster dose in mice.** "BNT162b2 WT" corresponds to a monovalent mRNA vaccine encoding a SARS-CoV-2 S protein of a Wuhan strain, "BNT162b2 Bivalent WT + BA.4/5" corresponds to a bivalent vaccine comprising an RNA encoding an S protein of a Wuhan strain and an RNA encoding a SARS-CoV-2 S protein of an Omicron BA.4/5 variant. On day 134, mice were administered a monovalent or bivalent vaccine encoding one of the strains, variants, or combinations indicated in the figure. On days 134 and 160, blood samples were collected (represented by the test tube racks).

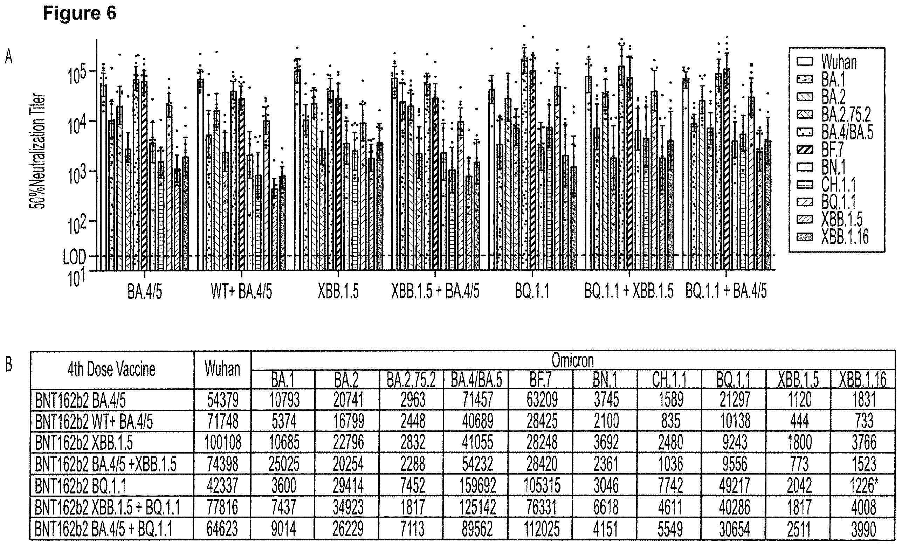

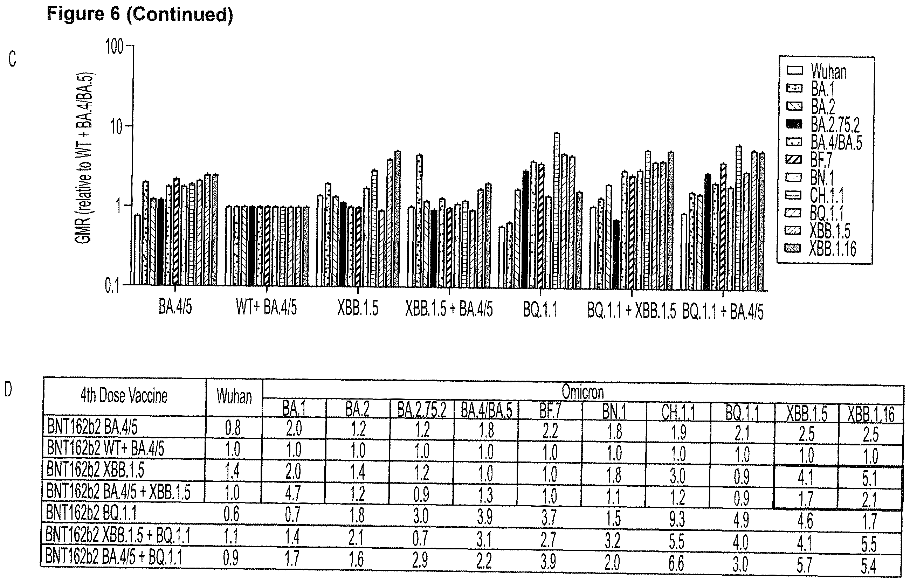

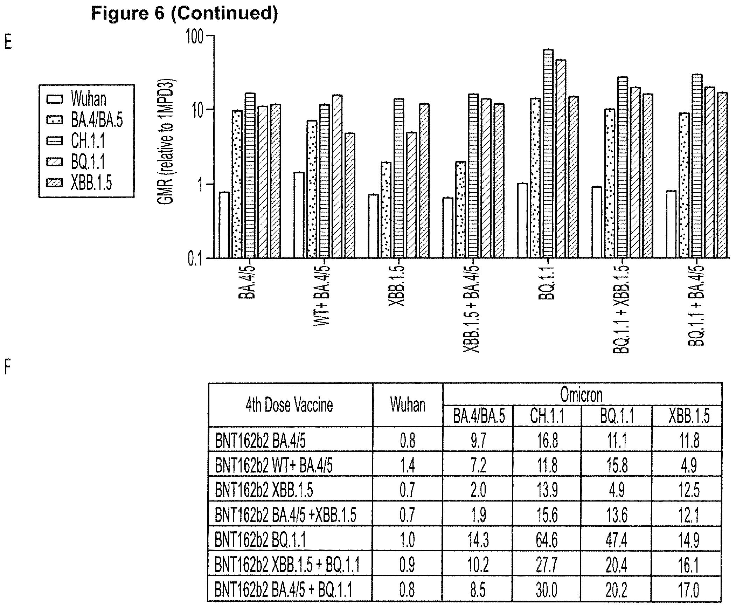

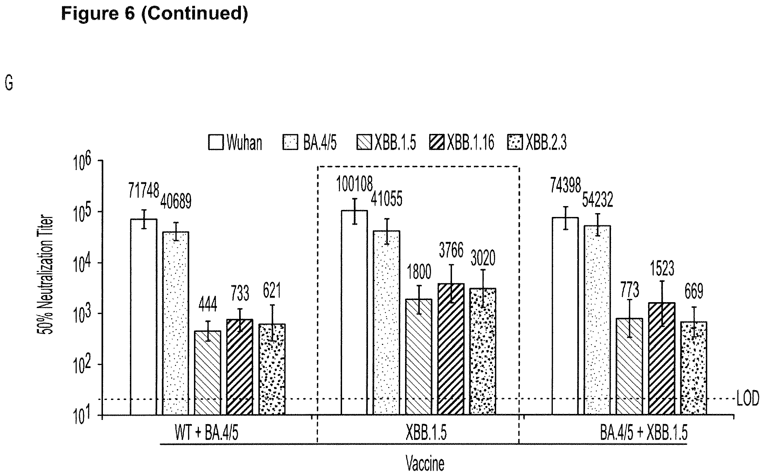

**Fig. 6****. An XBB.1.5-adapted booster vaccine elicits the highest neutralization titers against an XBB.1.5 and XBB.1.6 pseudovirus.** Shown are results collected in performing the experiment summarized in Figure 5. (A) shows neutralization titers collected for a panel of pseudoviruses (indicated in the figure legend). X-axis indicates vaccine administered. Titers collected at day 160. (B) shows neutralization titer values for the bar graph shown in panel (A). (C) Shows geometric mean ratios (GMR) of neutralization titers induced by the indicated vaccine relative to neutralization titers induced by a bivalent vaccine comprising an RNA encoding a SARS-CoV-2 S protein from a Wuhan strain (WT in the figure) and an RNA encoding a SARS-CoV-2 S protein from an Omicron BA.4/5 variant (GMR values calculated using neutralization titers at day 160). (D) provides GMR values for the bar graph shown in panel (C). (E) Shows GMR values comparing neutralization titers induced by the indicated vaccines relative to neutralization titers at day 135 (pre dose 4). (F) shows GMR values for the graph shown in panel (E). (G) shows neutralization titers against further SARS-CoV-2 variants (including XBB.2.3).

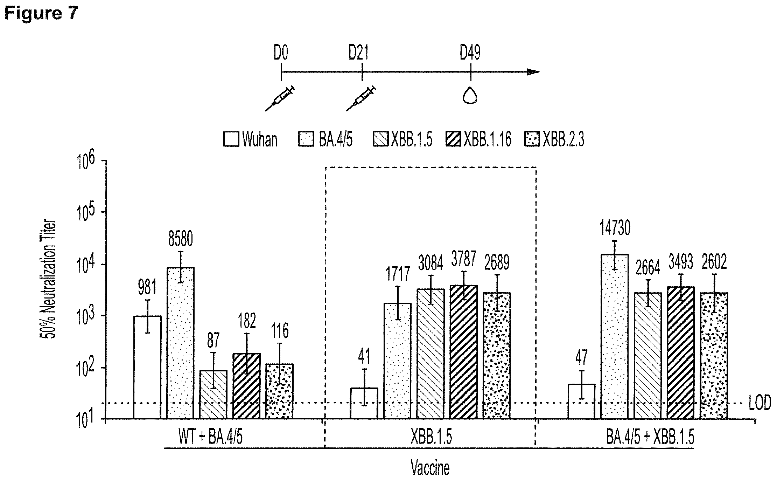

**Fig. 7****. Certain variant-adapted vaccines induce a neutralization response against Omicron XBB variants in vaccine-naïve mice.** A summary of the experimental protocol is depicted at the top of the figure (syringes indicate days on which candidate vaccines were administered and the droplet indicates a day on which blood was collected). Vaccine naive mice were administered two doses of the indicated candidate vaccines. Four weeks after receiving a second dose of vaccine, mice were bled and neutralization titers collected. As shown in the figure, neutralization titers were induced against each of the variants tested, with a monovalent XBB.1.5-adapted vaccine inducing the highest neutralization titers against each of the XBB variants tested.

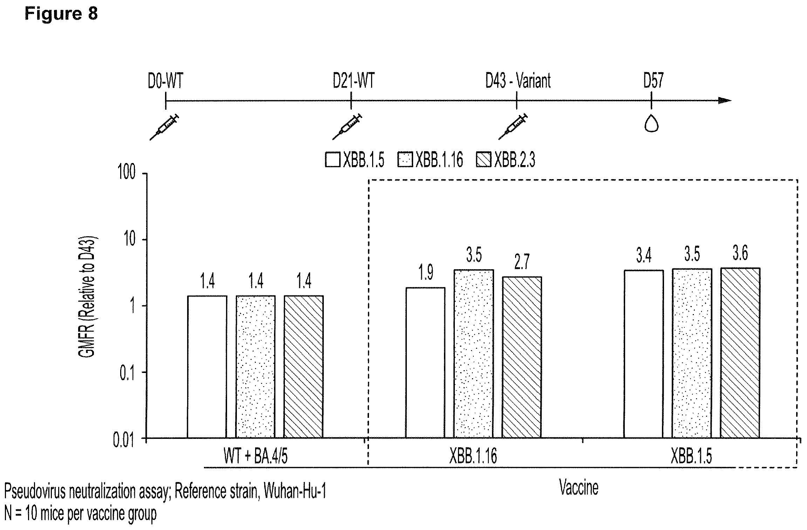

**Fig. 8****. Certain variant-adapted vaccines induce a neutralization response against Omicron XBB variants in vaccine-experienced mice.** A summary of the experimental protocol is depicted at the top of the figure (syringes indicate days on which candidate vaccines were administered and the droplet indicates a day on which blood was collected). Mice were administered two doses of RNA encoding a SARS-CoV-2 S protein of a Wuhan strain (WT), followed by a dose of a candidate vaccine. Two weeks after receiving a dose of candidate vaccine, mice were bled and neutralization titers collected. As shown in the figure, neutralization titers were induced against each of the variants tested, with a monovalent XBB.1.5-adapted vaccine inducing the highest neutralization titers against each of the XBB variants tested.

### Definitions

The term "about" means approximately or nearly, and in the context of a numerical value or range set forth herein in one embodiment means ± 20%, ± 10%, ± 5%, or ± 3% of the numerical value or range recited or claimed.

The terms "a" and "an" and "the" and similar reference used in the context of describing the present disclosure (especially in the context of the claims) are to be construed to cover both the singular and the plural, unless otherwise indicated herein or clearly contradicted by context. Recitation of ranges of values herein is merely intended to serve as a shorthand method of referring individually to each separate value falling within the range. Unless otherwise indicated herein, each individual value is incorporated into the specification as if it was individually recited herein. All methods described herein can be performed in any suitable order unless otherwise indicated herein or otherwise clearly contradicted by context. The use of any and all examples, or exemplary language (e.g., "such as"), provided herein is intended merely to better illustrate the disclosure and does not pose a limitation on the scope of the claims. No language in the specification should be construed as indicating any non-claimed element essential to the practice of the disclosure.

### RNA

In the present disclosure, the term "RNA" relates to a nucleic acid molecule which includes ribonucleotide residues. In preferred embodiments, the RNA contains all or a majority of ribonucleotide residues. As used herein, "ribonucleotide" refers to a nucleotide with a hydroxyl group at the 2'-position of a β-D-ribofuranosyl group. RNA encompasses without limitation, double stranded RNA, single stranded RNA, isolated RNA such as partially purified RNA, essentially pure RNA, synthetic RNA, recombinantly produced RNA, as well as modified RNA that differs from naturally occurring RNA by the addition, deletion, substitution and/or alteration of one or more nucleotides. Such alterations may refer to addition of non-nucleotide material to internal RNA nucleotides or to the end(s) of RNA. It is also contemplated herein that nucleotides in RNA may be non-standard nucleotides, such as chemically synthesized nucleotides or deoxynucleotides. For the present disclosure, these altered RNAs are considered analogs of naturally-occurring RNA.

In certain embodiments of the present disclosure, the RNA is messenger RNA (mRNA) that relates to a RNA transcript which encodes a peptide or protein. As established in the art, mRNA generally contains a 5' untranslated region (5'-UTR), a peptide coding region and a 3' untranslated region (3'-UTR). In some embodiments, the RNA is produced by *in vitro* transcription or chemical synthesis. In one embodiment, the mRNA is produced by *in vitro* transcription using a DNA template where DNA refers to a nucleic acid that contains deoxyribonucleotides.

In one embodiment, RNA described herein may have modified nucleosides. In some embodiments, RNA comprises a modified nucleoside in place of at least one (e.g., every) uridine. In some embodiments, one or more uridine in the RNA described herein is replaced by a modified nucleoside. In some embodiments, RNA comprises a modified nucleoside in place of each uridine. In some embodiments, the modified nucleoside is a modified uridine.

One exemplary modified nucleoside is N1-methyl-pseudouridine (m1ψ), which has the structure:

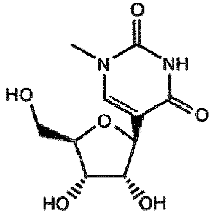

In some embodiments, RNA according to the present disclosure comprises a 5'-cap. The term "5'-cap" refers to a structure found on the 5'-end of an mRNA molecule and generally consists of a guanosine nucleotide connected to the mRNA via a 5'- to 5'-triphosphate linkage. In one embodiment, this guanosine is methylated at the 7-position. Providing an RNA with a 5'-cap or 5'-cap analog may be achieved by *in vitro* transcription, in which the 5'-cap is co-transcriptionally expressed into the RNA strand, or may be attached to RNA post-transcriptionally using capping enzymes.

In some embodiments, the building block cap for RNA is m₂^{7,3'-O}Gppp(m₁^{2'-O})ApG (also sometimes referred to as m₂^{7,3'-O}G(5')ppp(5')m^{2'-O}ApG), which has the following structure:

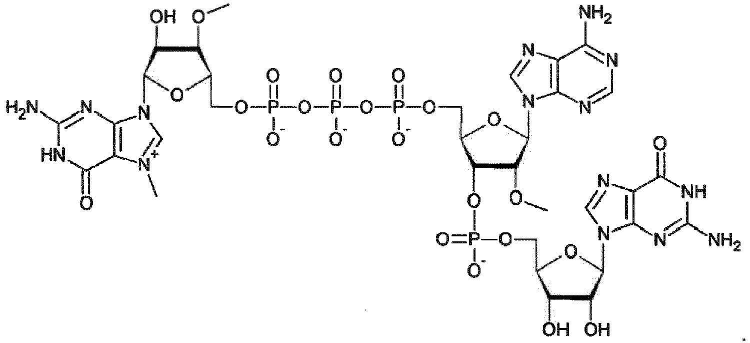

In one embodiment, the 5'-UTR sequence is derived from the human alpha-globin mRNA and optionally has an optimized 'Kozak sequence' to increase translational efficiency.

In one embodiment, RNA encoding an amino acid sequence comprising a SARS-CoV-2 S protein, an immunogenic variant thereof, or an immunogenic fragment of the SARS-CoV-2 S protein or the immunogenic variant thereof comprises a 5' UTR comprising the nucleotide sequence of SEQ ID NO: 12, or a nucleotide sequence having at least 99%, 98%, 97%, 96%, 95%, 90%, 85%, or 80% identity to the nucleotide sequence of SEQ ID NO: 12.

In one embodiment, a combination of two sequence elements (FI element) derived from the "amino terminal enhancer of split" (AES) mRNA (called F) and the mitochondrial encoded 12S ribosomal RNA (called I) are placed between the coding sequence and the poly(A) sequence to assure higher maximum protein levels and prolonged persistence of the RNA (e.g., mRNA). In one embodiment, two re-iterated 3'-UTRs derived from the human beta-globin mRNA are placed between the coding sequence and the poly(A) sequence to assure higher maximum protein levels and prolonged persistence of the RNA (e.g., mRNA).

In one embodiment, RNA encoding an amino acid sequence comprising a SARS-CoV-2 S protein, an immunogenic variant thereof, or an immunogenic fragment of the SARS-CoV-2 S protein or the immunogenic variant thereof comprises a 3' UTR comprising the nucleotide sequence of SEQ ID NO: 13, or a nucleotide sequence having at least 99%, 98%, 97%, 96%, 95%, 90%, 85%, or 80% identity to the nucleotide sequence of SEQ ID NO: 13.

In one embodiment, a poly(A) sequence measuring 110 nucleotides in length, consisting of a stretch of 30 adenosine residues, followed by a linker sequence (e.g., 10 nucleotide linker sequence) and another 70 adenosine residues is used. This poly(A) sequence was designed to enhance RNA stability and translational efficiency.

In one embodiment, the poly-A sequence comprises at least 100 nucleotides.

In one embodiment, the poly-A sequence comprises or consists of the nucleotide sequence of SEQ ID NO: 14.

### RNA Encoding SARS-CoV-2 S protein of an XBB.1.5 Variant

In some embodiments, RNA described herein encodes a SARS-CoV-2 S protein comprising one or more mutations characteristic of an Omicron XBB.1.5 variant. In some embodiments, the one or more mutations characteristic of an Omicron XBB.1.5 variant include F486P. In some embodiments, the one or more mutations characteristic of an Omicron XBB.1.5 variant include T19I, Δ24-26, A27S, V83A, G142D, Δ144, H146Q, Q183E, V213E, G252V, G339H, R346T, L368I, S371F, S373P, S375F, T376A, D405N, R408S, K417N, N440K, V445P, G446S, N460K, S477N, T478K, E484A, S486P, F490S, Q498R, N501Y, Y505H, D614G, H655Y, N679K, P681H, N764K, D796Y, Q954H, and N969K, or any combination thereof. In some embodiments, the one or more mutations characteristic of an Omicron XBB.1.5 variant include T19I, Δ24-26, A27S, V83A, G142D, Δ145, H146Q, Q183E, V213E, G252V, G339H, R346T, L368I, S371F, S373P, S375F, T376A, D405N, R408S, K417N, N440K, V445P, G446S, N460K, S477N, T478K, E484A, F486P, F490S, Q498R, N501Y, Y505H, D614G, H655Y, N679K, P681H, N764K, D796Y, Q954H, and N969K, or any combination thereof.

In some embodiments, RNA described herein encodes a SARS-CoV-2 S protein comprising one more mutations that stabilize a prefusion confirmation. In some embodiments, stabilization of a prefusion conformation of a SARS-CoV-2 S protein may be obtained by introducing two consecutive proline substitutions at residues 986 and 987 in the full length spike protein (positions shown relative to SEQ ID NO: 1; for variant adapted sequences, proline substitutions can be introduced at corresponding positions). Specifically, spike (S) protein stabilized protein variants can be obtained by exchanging the amino acid residue at position 986 to proline and the amino acid residue at position 987 to proline. In one embodiment, a SARS-CoV-2 S protein variant wherein the prototypical prefusion conformation is stabilized comprises the amino acid sequence shown in SEQ ID NO: 7. In one embodiment, the SARS-CoV-2 S protein can comprise the following mutations relative to SEQ ID NO: 7: T19I, Δ24-26, A27S, V83A, G142D, Δ145, H146Q, Q183E, V213E, G252V, G339H, R346T, L368I, S371F, S373P, S375F, T376A, D405N, R408S, K417N, N440K, V445P, G446S, N460K, S477N, T478K, E484A, F486P, F490S, Q498R, N501Y, Y505H, D614G, H655Y, N679K, P681H, N764K, D796Y, Q954H, and N969K.

In some embodiments, an RNA composition described herein comprises an RNA encoding a polypeptide as set forth in SEQ ID NO: 158 or an immunogenic fragment thereof, or a variant thereof (e.g., having at least 70% or more, including, e.g., at least 80%, at least 85%, at least 90%, at least 95%, at least 96%, at least 97%, at least 98%, or higher, identity to SEQ ID NO: 158). In some embodiments, an RNA composition comprises an RNA that includes the sequence of SEQ ID NO: 159 or a variant thereof (e.g., having at least 70% or more, including, e.g., at least 80%, at least 85%, at least 90%, at least 95%, at least 96%, at least 97%, at least 98%, or higher, identity to SEQ ID NO: 159). In some embodiments, an RNA composition comprises an RNA that includes the sequence of SEQ ID NO: 161 or a variant thereof (e.g., having at least 70% or more, including, e.g., at least 80%, at least 85%, at least 90%, at least 95%, at least 96%, at least 97%, at least 98%, or higher, identity to SEQ ID NO: 161).

In some embodiments, an RNA composition comprises an RNA comprising (a) a nucleotide sequence of SEQ ID NO: 161 or a sequence that is at least 70% identical to SEQ ID NO: 161 (e.g., at least 80%, at least 85%, at least 90%, at least 95%, at least 96%, at least 97%, at least 98%, at least 99% or higher, identity to SEQ ID NO: 161), and/or (b) a nucleotide sequence that encodes a SARS-CoV-2 S protein having an amino acid sequence of SEQ ID NO: 158, or an amino acid sequence that is at least 70% identical to SEQ ID NO: 158 (e.g., at least 80%, at least 85%, at least 90%, at least 95%, at least 96%, at least 97%, at least 98%, at least 99% or higher, identity to SEQ ID NO: 158), and (c) wherein the SARS-CoV-2 S protein optionally comprises one or more mutations that stabilize a prefusion confirmation (e.g., proline mutations at positions 982 and 983 of SEQ ID NO: 158). In some embodiments, such a composition comprises one or more additional RNAs, each encoding an S protein of a non-XBB.1.5 SARS-CoV strain or variant (e.g., a Wuhan strain, an Omicron BA.4/5 variant, and/or an Omicron BA.2.75.2 variant (e.g., such RNAs described herein)).

In some embodiments, an RNA composition comprises an RNA comprising a nucleotide sequence that encodes a SARS-CoV-2 S protein comprising one or more mutations characteristic of an Omicron XBB.1.5 variant and wherein:
a) the SARS-CoV-2 S protein comprises an amino acid sequence having at least 99%, 98%, 97%, 96%, 95%, 90%, 85%, or 80% identity to SEQ ID NO: 158, or an immunogenic fragment thereof; and/or
b) the RNA molecule encoding the SARS-CoV-2 S protein comprises a nucleotide sequence having at least 99%, 98%, 97%, 96%, 95%, 90%, 85%, or 80% identity to SEQ ID NO: 159; and/or at least 99%, 98%, 97%, 96%, 95%, 90%, 85%, or 80% identity to SEQ ID NO: 161, and wherein
c) the one or more mutations characteristic of an Omicron XBB.1.5 variant are optionally selected from: T19I, Δ24-26, A27S, V83A, G142D, Δ145, H146Q, Q183E, V213E, G252V, G339H, R346T, L368I, S371F, S373P, S375F, T376A, D405N, R408S, K417N, N440K, V445P, G446S, N460K, S477N, T478K, E484A, F486P, F490S, Q498R, N501Y, Y505H, D614G, H655Y, N679K, P681H, N764K, D796Y, Q954H, and N969K, or any combination thereof.

In some embodiments, a composition comprising an RNA that comprises a nucleotide sequence encoding a SARS-CoV-2 S protein comprising one or more mutations characteristic of an XBB.1.5 variant further comprises one or more additional RNAs, each having a nucleotide sequence encoding a SARS-CoV-2 S protein of a non-XBB.1.5 SARS-CoV-2 strain or variant or strain. In some embodiments, such non-XBB.1.5 strains or variants include a Wuhan strain, an Omicron BA.4/5 strain, and/or a BQ.1.1 variant. In some embodiments, the one or more additional RNA molecules comprise a sequence that is at least 70% identical (e.g., at least 80%, at least 85%, at least 90%, at least 95%, at least 96%, at least 97%, at least 98%, at least 99% or higher, identity to) SEQ ID NO: 20, 70, or 103, or wherein the one or more additional RNA molecules comprise a sequence SEQ ID NO: 20, 72, or 103.

**Table 1. Sequence of one embodiment of an exemplary Omicron XBB.1.5-specific RNA vaccine**

| SEQ ID NO. | Brief Description | Sequence |
|---|---|---|
| 158 | Amino acid sequence of RNA-encoded SARS-CoV-2 S protein from an Omicron XBB.1.5 variant (with PRO mutations at positions corresponding to K986P and V987P of SEQ ID NO: 1; i.e., PRO mutations at positions 982 and 983 of SEQ ID NO: 158) | MFVFLVLLPLVSSQCVNLITRTQSYTNSFTRGVYYPDKVFRSSVLHSTQDLFLPFFSNVTWFHAIHVSGTNGTKR FDNPALPFNDGVYFASTEKSNIIRGWIFGTTLDSKTQSLLIVNNATNVVIKVCEFQFCNDPFLDVYQKNNKSWM ESEFRVYSSANNCTFEYVSQPFLMDLEGKEGNFKNLREFVFKNIDGYFKIYSKHTPINLERDLPQGFSALEPLVDL PIGINITRFQTLLALHRSYLTPVDSSSGWTAGAAAYYVGYLQPRTFLLKYNENGTITDAVDCALDPLSETKCTLKS FTVEKGIYQTSNFRVQPTESIVRFPNITNLCPFHEVFNATTFASVYAWNRKRISNCVADYSVIYNFAPFFAFKCYG VSPTKLNDLCFTNVYADSFVIRGNEVSQIAPGQTGNIADYNYKLPDDFTGCVIAWNSNKLDSKPSGNYNYLYRL FRKSKLKPFERDISTEIYQAGNKPCNGVAGPNCYSPLQSYGFRPTYGVGHQPYRVVVLSFELLHAPATVCGPKKS TNLVKNKCVNFNFNGLTGTGVLTESNKKFLPFQQFGRDIADTTDAVRDPQTLEILDITPCSFGGVSVITPGTNTS NQVAVLYQGVNCTEVPVAIHADQLTPTWRVYSTGSNVFQTRAGCLIGAEYVNNSYECDIPIGAGICASYQTQTK SHRRARSVASQSIIAYTMSLGAENSVAYSNNSIAIPTNFTISVTTEILPVSMTKTSVDCTMYICGDSTECSNLLLQ YGSFCTQLKRALTGIAVEQDKNTQEVFAQVKQIYKTPPIKYFGGFNFSQILPDPSKPSKRSFIEDLLFNKVTLADA GFIKQYGDCLGDIAARDLICAQKFNGLTVLPPLLTDEMIAQYTSALLAGTITSGWTFGAGAALQIPFAMQMAYRF NGIGVTQNVLYENQKLIANQFNSAIGKIQDSLSSTASALGKLQDVVNHNAQALNTLVKQLSSKFGAISSVLNDIL SRLDPPEAEVQIDRLITGRLQSLQTYVTQQLIRAAEIRASANLAATKMSECVLGQSKRVDFCGKGYHLMSFPQSA PHGVVFLHVTYVPAQEKNFTTAPAICHDGKAHFPREGVFVSNGTHWFVTQRNFYEPQIITTDNTFVSGNCDVVI GIVNNTVYDPLQPELDSFKEELDKYFKNHTSPDVDLGDISGINASVVNIQKEIDRLNEVAKNLNESLIDLQELGKY EQYIKWPWYIWLGFIAGLIAIVMVTIMLCCMTSCCSCLKGCCSCGSCCKFDEDDSEPVLKGVKLHYT |
| 159 | RNA sequence encoding a SARS-CoV-2 S protein from an Omicron XBB.1.5 variant | auguucguguuccuggugcugcugccucugguguccagccagugugugaaccugaucaccagaacacagucauacaccaacagc uuuaccagaggcguguacuaccccgacaagguguucagauccagcgugcugcacucuacccaggaccuguuccugccuuucuuca gcaacgugaccugguuccacgccauccacguguccggcaccaauggcaccaagagauucgacaaccccgcccugcccuucaacgac gggguguacuuugccagcaccgagaaguccaacaucaucagaggcuggaucuucggcaccacacuggacagcaagacccagagcc ugcugaucgugaacaacgccaccaacguggucaucaaagugugcgaguuccaguucugcaacgaccccuuccuggacgucuacca gaagaacaacaagagcuggauggaaagcgaguuccggguguacagcagcgccaacaacugcaccuucgaguacgugucccagccu uuccugauggaccuggaaggcaaggagggcaacuucaagaaccugcgcgaguucguguuuaagaacaucgacggcuacuucaag aucuacagcaagcacaccccuaucaaccucgagcgggaucugccucagggcuucucugcucuggaaccccugguggaucugccca ucggcaucaacaucacccgguuucagacacugcuggcccugcacagaagcuaccugacaccuguggauagcagcagcggauggac |
| | | agcuggugccgccgcuuacuaugugggcuaccugcagccuagaaccuuccugcugaaguacaacgagaacggcaccaucaccgac gccguggauugugcucuggauccucugagcgagacaaagugcacccugaaguccuucaccguggaaaagggcaucuaccagacca gcaacuuccgggugcagcccaccgaauccaucgugcgguuccccaauaucaccaaucugugccccuuccacgagguguucaaugcc accaccuucgccucuguguacgccuggaaccggaagcggaucagcaauugcguggccgacuacuccgugaucuacaacuucgccc ccuucuucgcauucaagugcuacggcguguccccuaccaagcugaacgaccugugcuucacaaacguguacgccgacagcuucgu gauccggggaaacgaagugucacagauugccccuggacagacaggcaacaucgccgacuacaacuacaagcugcccgacgacuuc accggcugugugauugccuggaacagcaacaagcuggacuccaaacccagcggcaacuacaauuaccuguaccggcuguuccgga aguccaagcugaagcccuucgagcgggacaucuccaccgagaucuaucaggccggcaacaagccuuguaacggcguggcaggccc caacugcuacagcccacugcaguccuacggcuuuaggcccacauacggcgugggccaccagcccuacagagugguggugcugagc uucgaacugcugcaugccccugccacagugugcggcccuaagaaaagcaccaaucucgugaagaacaaaugcgugaacuucaacu ucaacggccugaccggcaccggcgugcugacagagagcaacaagaaguuccugccauuccagcaguuuggccgggauaucgccga uaccacagacgccguuagagauccccagacacuggaaauccuggacaucaccccuugcagcuucggcggagugucugugaucacc ccuggcaccaacaccagcaaucagguggcagugcuguaccagggcgugaacuguaccgaagugcccguggccauucacgccgauc agcugacaccuacauggcggguguacuccaccggcagcaauguguuucagaccagagccggcugucugaucggagccgaguacg ugaacaauagcuacgagugcgacauccccaucggcgcuggaaucugcgccagcuaccagacacagacaaagagccaccggagagc cagaagcguggccagccagagcaucauugccuacacaaugucucugggcgccgagaacagcguggccuacuccaacaacucuauc gcuauccccaccaacuucaccaucagcgugaccacagagauccugccuguguccaugaccaagaccagcguggacugcaccaugua caucugcggcgauuccaccgagugcuccaaccugcugcugcaguacggcagcuucugcacccagcugaaaagagcccugacaggg aucgccguggaacaggacaagaacacccaagagguguucgcccaagugaagcagaucuacaagaccccuccuaucaaguacuucg gcggcuucaauuucagccagauucugcccgauccuagcaagcccagcaagcggagcuucaucgaggaccugcuguucaacaaagu gacacuggccgacgccggcuucaucaagcaguauggcgauugucugggcgacauugccgccagggaucugauuugcgcccagaa guuuaacggacugacagugcugccuccucugcugaccgaugagaugaucgcccaguacacaucugcccugcuggccggcacaauc acaagcggcuggacauuuggagcaggcgccgcucugcagauccccuuugcuaugcagauggccuaccgguucaacggcaucgga gugacccagaaugugcuguacgagaaccagaagcugaucgccaaccaguucaacagcgccaucggcaagauccaggacagccuga gcagcacagcaagcgcccugggaaagcugcaggacguggucaaccacaaugcccaggcacugaacacccuggucaagcagcugucc uccaaguucggcgccaucagcucugugcugaacgauauccugagcagacuggacccuccugaggccgaggugcagaucgacagac ugaucacaggcagacugcagagccuccagacauacgugacccagcagcugaucagagccgccgagauuagagccucugccaaucu ggccgccaccaagaugucugagugugugcugggccagagcaagagaguggacuuuugcggcaagggcuaccaccugaugagcuu |
| | | cccucagucugccccucacggcgugguguuucugcacgugacauaugugcccgcucaagagaagaauuucaccaccgcuccagcc aucugccacgacggcaaagcccacuuuccuagagaaggcguguucguguccaacggcacccauugguucgugacacagcggaacu ucuacgagccccagaucaucaccaccgacaacaccuucgugucuggcaacugcgacgucgugaucggcauugugaacaauaccgu guacgacccucugcagcccgagcuggacagcuucaaagaggaacuggacaaguacuuuaagaaccacacaagccccgacguggac cugggcgauaucagcggaaucaaugccagcgucgugaacauccagaaagagaucgaccggcugaacgagguggccaagaaucug aacgagagccugaucgaccugcaagaacuggggaaguacgagcaguacaucaaguggcccugguacaucuggcugggcuuuauc gccggacugauugccaucgugauggucacaaucaugcuguguugcaugaccagcugcuguagcugccugaagggcuguuguagc uguggcagcugcugcaaguucgacgaggacgauucugagcccgugcugaagggcgugaaacugcacuacacaugauga |
| 160 | DNA sequence encoding a SARS-CoV-2 S protein from an Omicron XBB.1.5 variant | atgttcgtgttcctggtgctgctgcctctggtgtccagccagtgtgtgaacctgatcaccagaacacagtcatacaccaacagctttaccagagg cgtgtactaccccgacaaggtgttcagatccagcgtgctgcactctacccaggacctgttcctgcctttcttcagcaacgtgacctggttccacgc catccacgtgtccggcaccaatggcaccaagagattcgacaaccccgccctgcccttcaacgacggggtgtactttgccagcaccgagaagt ccaacatcatcagaggctggatcttcggcaccacactggacagcaagacccagagcctgctgatcgtgaacaacgccaccaacgtggtcatc aaagtgtgcgagttccagttctgcaacgaccccttcctggacgtctaccagaagaacaacaagagctggatggaaagcgagttccgggtgta cagcagcgccaacaactgcaccttcgagtacgtgtcccagcctttcctgatggacctggaaggcaaggagggcaacttcaagaacctgcgcg agttcgtgtttaagaacatcgacggctacttcaagatctacagcaagcacacccctatcaacctcgagcgggatctgcctcagggcttctctgct ctggaacccctggtggatctgcccatcggcatcaacatcacccggtttcagacactgctggccctgcacagaagctacctgacacctgtggat agcagcagcggatggacagctggtgccgccgcttactatgtgggctacctgcagcctagaaccttcctgctgaagtacaacgagaacggcac catcaccgacgccgtggattgtgctctggatcctctgagcgagacaaagtgcaccctgaagtccttcaccgtggaaaagggcatctaccagac cagcaacttccgggtgcagcccaccgaatccatcgtgcggttccccaatatcaccaatctgtgccccttccacgaggtgttcaatgccaccacct tcgcctctgtgtacgcctggaaccggaagcggatcagcaattgcgtggccgactactccgtgatctacaacttcgcccccttcttcgcattcaag tgctacggcgtgtcccctaccaagctgaacgacctgtgcttcacaaacgtgtacgccgacagcttcgtgatccggggaaacgaagtgtcaca gattgcccctggacagacaggcaacatcgccgactacaactacaagctgcccgacgacttcaccggctgtgtgattgcctggaacagcaaca agctggactccaaacccagcggcaactacaattacctgtaccggctgttccggaagtccaagctgaagcccttcgagcgggacatctccacc gagatctatcaggccggcaacaagccttgtaacggcgtggcaggccccaactgctacagcccactgcagtcctacggctttaggcccacata cggcgtgggccaccagccctacagagtggtggtgctgagcttcgaactgctgcatgcccctgccacagtgtgcggccctaagaaaagcacc aatctcgtgaagaacaaatgcgtgaacttcaacttcaacggcctgaccggcaccggcgtgctgacagagagcaacaagaagttcctgccatt ccagcagtttggccgggatatcgccgataccacagacgccgttagagatccccagacactggaaatcctggacatcaccccttgcagcttcgg cggagtgtctgtgatcacccctggcaccaacaccagcaatcaggtggcagtgctgtaccagggcgtgaactgtaccgaagtgcccgtggcca |
| | | ttcacgccgatcagctgacacctacatggcgggtgtactccaccggcagcaatgtgtttcagaccagagccggctgtctgatcggagccgagt acgtgaacaatagctacgagtgcgacatccccatcggcgctggaatctgcgccagctaccagacacagacaaagagccaccggagagcca gaagcgtggccagccagagcatcattgcctacacaatgtctctgggcgccgagaacagcgtggcctactccaacaactctatcgctatcccca ccaacttcaccatcagcgtgaccacagagatcctgcctgtgtccatgaccaagaccagcgtggactgcaccatgtacatctgcggcgattcca ccgagtgctccaacctgctgctgcagtacggcagcttctgcacccagctgaaaagagccctgacagggatcgccgtggaacaggacaagaa cacccaagaggtgttcgcccaagtgaagcagatctacaagacccctcctatcaagtacttcggcggcttcaatttcagccagattctgcccgat cctagcaagcccagcaagcggagcttcatcgaggacctgctgttcaacaaagtgacactggccgacgccggcttcatcaagcagtatggcg attgtctgggcgacattgccgccagggatctgatttgcgcccagaagtttaacggactgacagtgctgcctcctctgctgaccgatgagatgat cgcccagtacacatctgccctgctggccggcacaatcacaagcggctggacatttggagcaggcgccgctctgcagatcccctttgctatgca gatggcctaccggttcaacggcatcggagtgacccagaatgtgctgtacgagaaccagaagctgatcgccaaccagttcaacagcgccatc ggcaagatccaggacagcctgagcagcacagcaagcgccctgggaaagctgcaggacgtggtcaaccacaatgcccaggcactgaacac cctggtcaagcagctgtcctccaagttcggcgccatcagctctgtgctgaacgatatcctgagcagactggaccctcctgaggccgaggtgca gatcgacagactgatcacaggcagactgcagagcctccagacatacgtgacccagcagctgatcagagccgccgagattagagcctctgcc aatctggccgccaccaagatgtctgagtgtgtgctgggccagagcaagagagtggacttttgcggcaagggctaccacctgatgagcttccct cagtctgcccctcacggcgtggtgtttctgcacgtgacatatgtgcccgctcaagagaagaatttcaccaccgctccagccatctgccacgacg gcaaagcccactttcctagagaaggcgtgttcgtgtccaacggcacccattggttcgtgacacagcggaacttctacgagccccagatcatca ccaccgacaacaccttcgtgtctggcaactgcgacgtcgtgatcggcattgtgaacaataccgtgtacgaccctctgcagcccgagctggaca gcttcaaagaggaactggacaagtactttaagaaccacacaagccccgacgtggacctgggcgatatcagcggaatcaatgccagcgtcgt gaacatccagaaagagatcgaccggctgaacgaggtggccaagaatctgaacgagagcctgatcgacctgcaagaactggggaagtacg agcagtacatcaagtggccctggtacatctggctgggctttatcgccggactgattgccatcgtgatggtcacaatcatgctgtgttgcatgacc agctgctgtagctgcctgaagggctgttgtagctgtggcagctgctgcaagttcgacgaggacgattctgagcccgtgctgaagggcgtgaa actgcactacacatgatga |
| 161 | Full length RNA construct encoding a SARS-CoV-2 S protein from an Omicron XBB.1.5 variant | Agaauaaacuaguauucuucugguccccacagacucagagagaacccgccaccauguucguguuccuggugcugcugccucugg uguccagccagugugugaaccugaucaccagaacacagucauacaccaacagcuuuaccagaggcguguacuaccccgacaaggu guucagauccagcgugcugcacucuacccaggaccuguuccugccuuucuucagcaacgugaccugguuccacgccauccacgug uccggcaccaauggcaccaagagauucgacaaccccgcccugcccuucaacgacgggguguacuuugccagcaccgagaaguccaa caucaucagaggcuggaucuucggcaccacacuggacagcaagacccagagccugcugaucgugaacaacgccaccaacgugguc aucaaagugugcgaguuccaguucugcaacgaccccuuccuggacgucuaccagaagaacaacaagagcuggauggaaagcgag |
| | | uuccggguguacagcagcgccaacaacugcaccuucgaguacgugucccagccuuuccugauggaccuggaaggcaaggagggca acuucaagaaccugcgcgaguucguguuuaagaacaucgacggcuacuucaagaucuacagcaagcacaccccuaucaaccucga gcgggaucugccucagggcuucucugcucuggaaccccugguggaucugcccaucggcaucaacaucacccgguuucagacacug cuggcccugcacagaagcuaccugacaccuguggauagcagcagcggauggacagcuggugccgccgcuuacuaugugggcuacc ugcagccuagaaccuuccugcugaaguacaacgagaacggcaccaucaccgacgccguggauugugcucuggauccucugagcga gacaaagugcacccugaaguccuucaccguggaaaagggcaucuaccagaccagcaacuuccgggugcagcccaccgaauccaucg ugcgguuccccaauaucaccaaucugugccccuuccacgagguguucaaugccaccaccuucgccucuguguacgccuggaaccgg aagcggaucagcaauugcguggccgacuacuccgugaucuacaacuucgcccccuucuucgcauucaagugcuacggcguguccc cuaccaagcugaacgaccugugcuucacaaacguguacgccgacagcuucgugauccggggaaacgaagugucacagauugcccc uggacagacaggcaacaucgccgacuacaacuacaagcugcccgacgacuucaccggcugugugauugccuggaacagcaacaag cuggacuccaaacccagcggcaacuacaauuaccuguaccggcuguuccggaaguccaagcugaagcccuucgagcgggacaucu ccaccgagaucuaucaggccggcaacaagccuuguaacggcguggcaggccccaacugcuacagcccacugcaguccuacggcuuu aggcccacauacggcgugggccaccagcccuacagagugguggugcugagcuucgaacugcugcaugccccugccacagugugcg gcccuaagaaaagcaccaaucucgugaagaacaaaugcgugaacuucaacuucaacggccugaccggcaccggcgugcugacaga gagcaacaagaaguuccugccauuccagcaguuuggccgggauaucgccgauaccacagacgccguuagagauccccagacacug gaaauccuggacaucaccccuugcagcuucggcggagugucugugaucaccccuggcaccaacaccagcaaucagguggcagugc uguaccagggcgugaacuguaccgaagugcccguggccauucacgccgaucagcugacaccuacauggcggguguacuccaccgg cagcaauguguuucagaccagagccggcugucugaucggagccgaguacgugaacaauagcuacgagugcgacauccccaucggc gcuggaaucugcgccagcuaccagacacagacaaagagccaccggagagccagaagcguggccagccagagcaucauugccuacac aaugucucugggcgccgagaacagcguggccuacuccaacaacucuaucgcuauccccaccaacuucaccaucagcgugaccacag agauccugccuguguccaugaccaagaccagcguggacugcaccauguacaucugcggcgauuccaccgagugcuccaaccugcu gcugcaguacggcagcuucugcacccagcugaaaagagcccugacagggaucgccguggaacaggacaagaacacccaagaggug uucgcccaagugaagcagaucuacaagaccccuccuaucaaguacuucggcggcuucaauuucagccagauucugcccgauccua gcaagcccagcaagcggagcuucaucgaggaccugcuguucaacaaagugacacuggccgacgccggcuucaucaagcaguaugg cgauugucugggcgacauugccgccagggaucugauuugcgcccagaaguuuaacggacugacagugcugccuccucugcugac cgaugagaugaucgcccaguacacaucugcccugcuggccggcacaaucacaagcggcuggacauuuggagcaggcgccgcucug cagauccccuuugcuaugcagauggccuaccgguucaacggcaucggagugacccagaaugugcuguacgagaaccagaagcug aucgccaaccaguucaacagcgccaucggcaagauccaggacagccugagcagcacagcaagcgcccugggaaagcugcaggacg |
| | | uggucaaccacaaugcccaggcacugaacacccuggucaagcagcuguccuccaaguucggcgccaucagcucugugcugaacga uauccugagcagacuggacccuccugaggccgaggugcagaucgacagacugaucacaggcagacugcagagccuccagacauac gugacccagcagcugaucagagccgccgagauuagagccucugccaaucuggccgccaccaagaugucugagugugugcugggcc agagcaagagaguggacuuuugcggcaagggcuaccaccugaugagcuucccucagucugccccucacggcgugguguuucugc acgugacauaugugcccgcucaagagaagaauuucaccaccgcuccagccaucugccacgacggcaaagcccacuuuccuagagaa ggcguguucguguccaacggcacccauugguucgugacacagcggaacuucuacgagccccagaucaucaccaccgacaacaccu ucgugucuggcaacugcgacgucgugaucggcauugugaacaauaccguguacgacccucugcagcccgagcuggacagcuucaa agaggaacuggacaaguacuuuaagaaccacacaagccccgacguggaccugggcgauaucagcggaaucaaugccagcgucgu gaacauccagaaagagaucgaccggcugaacgagguggccaagaaucugaacgagagccugaucgaccugcaagaacuggggaa guacgagcaguacaucaaguggcccugguacaucuggcugggcuuuaucgccggacugauugccaucgugaugguacacaaucau gcuguguugcaugaccagcugcuguagcugccugaagggcuguuguagcuguggcagcugcugcaaguucgacgaggacgauu cugagcccgugcugaagggcgugaaacugcacuacacaugaugacucgagcugguacugcaugcacgcaaugcuagcugccccuu ucccguccuggguaccccgagucucccccgaccucgggucccagguaugcucccaccuccaccugccccacucaccaccucugcuag uuccagacaccucccaagcacgcagcaaugcagcucaaaacgcuuagccuagccacacccccacgggaaacagcagugauuaaccu uuagcaauaaacgaaaguuuaacuaagcuauacuaaccccaggguuggucaauuucgugccagccacacccuggagcuagcaaa aaaaaaaaaaaaaaaaaaaaaaaaaaagcauaugacuaaaaaaaaaaaaaaaaaaaaaaaaaaaaaaaaaaaaaaaaaaaaaaaaa aaaaaaaaaaaaaaaaaaaaaa |
| 162 | Full length DNA construct encoding a SARS-CoV-2 S protein from an Omicron XBB.1.5 variant | Agaataaactagtattcttctggtccccacagactcagagagaacccgccaccatgttcgtgttcctggtgctgctgcctctggtgtccagccag tgtgtgaacctgatcaccagaacacagtcatacaccaacagctttaccagaggcgtgtactaccccgacaaggtgttcagatccagcgtgctg cactctacccaggacctgttcctgcctttcttcagcaacgtgacctggttccacgccatccacgtgtccggcaccaatggcaccaagagattcga caaccccgccctgcccttcaacgacggggtgtactttgccagcaccgagaagtccaacatcatcagaggctggatcttcggcaccacactgga cagcaagacccagagcctgctgatcgtgaacaacgccaccaacgtggtcatcaaagtgtgcgagttccagttctgcaacgaccccttcctgga cgtctaccagaagaacaacaagagctggatggaaagcgagttccgggtgtacagcagcgccaacaactgcaccttcgagtacgtgtcccag cctttcctgatggacctggaaggcaaggagggcaacttcaagaacctgcgcgagttcgtgtttaagaacatcgacggctacttcaagatctac agcaagcacacccctatcaacctcgagcgggatctgcctcagggcttctctgctctggaacccctggtggatctgcccatcggcatcaacatca cccggtttcagacactgctggccctgcacagaagctacctgacacctgtggatagcagcagcggatggacagctggtgccgccgcttactatg tgggctacctgcagcctagaaccttcctgctgaagtacaacgagaacggcaccatcaccgacgccgtggattgtgctctggatcctctgagcg agacaaagtgcaccctgaagtccttcaccgtggaaaagggcatctaccagaccagcaacttccgggtgcagcccaccgaatccatcgtgcg |
| | | gttccccaatatcaccaatctgtgccccttccacgaggtgttcaatgccaccaccttcgcctctgtgtacgcctggaaccggaagcggatcagca attgcgtggccgactactccgtgatctacaacttcgcccccttcttcgcattcaagtgctacggcgtgtcccctaccaagctgaacgacctgtgct tcacaaacgtgtacgccgacagcttcgtgatccggggaaacgaagtgtcacagattgcccctggacagacaggcaacatcgccgactacaa ctacaagctgcccgacgacttcaccggctgtgtgattgcctggaacagcaacaagctggactccaaacccagcggcaactacaattacctgta ccggctgttccggaagtccaagctgaagcccttcgagcgggacatctccaccgagatctatcaggccggcaacaagccttgtaacggcgtgg caggccccaactgctacagcccactgcagtcctacggctttaggcccacatacggcgtgggccaccagccctacagagtggtggtgctgagc ttcgaactgctgcatgcccctgccacagtgtgcggccctaagaaaagcaccaatctcgtgaagaacaaatgcgtgaacttcaacttcaacggc ctgaccggcaccggcgtgctgacagagagcaacaagaagttcctgccattccagcagtttggccgggatatcgccgataccacagacgccg ttagagatccccagacactggaaatcctggacatcaccccttgcagcttcggcggagtgtctgtgatcacccctggcaccaacaccagcaatc aggtggcagtgctgtaccagggcgtgaactgtaccgaagtgcccgtggccattcacgccgatcagctgacacctacatggcgggtgtactcc accggcagcaatgtgtttcagaccagagccggctgtctgatcggagccgagtacgtgaacaatagctacgagtgcgacatccccatcggcgc tggaatctgcgccagctaccagacacagacaaagagccaccggagagccagaagcgtggccagccagagcatcattgcctacacaatgtc tctgggcgccgagaacagcgtggcctactccaacaactctatcgctatccccaccaacttcaccatcagcgtgaccacagagatcctgcctgtg tccatgaccaagaccagcgtggactgcaccatgtacatctgcggcgattccaccgagtgctccaacctgctgctgcagtacggcagcttctgc acccagctgaaaagagccctgacagggatcgccgtggaacaggacaagaacacccaagaggtgttcgcccaagtgaagcagatctacaa gacccctcctatcaagtacttcggcggcttcaatttcagccagattctgcccgatcctagcaagcccagcaagcggagcttcatcgaggacctg ctgttcaacaaagtgacactggccgacgccggcttcatcaagcagtatggcgattgtctgggcgacattgccgccagggatctgatttgcgcc cagaagtttaacggactgacagtgctgcctcctctgctgaccgatgagatgatcgcccagtacacatctgccctgctggccggcacaatcaca agcggctggacatttggagcaggcgccgctctgcagatcccctttgctatgcagatggcctaccggttcaacggcatcggagtgacccagaat gtgctgtacgagaaccagaagctgatcgccaaccagttcaacagcgccatcggcaagatccaggacagcctgagcagcacagcaagcgcc ctgggaaagctgcaggacgtggtcaaccacaatgcccaggcactgaacaccctggtcaagcagctgtcctccaagttcggcgccatcagctc tgtgctgaacgatatcctgagcagactggaccctcctgaggccgaggtgcagatcgacagactgatcacaggcagactgcagagcctccag acatacgtgacccagcagctgatcagagccgccgagattagagcctctgccaatctggccgccaccaagatgtctgagtgtgtgctgggcca gagcaagagagtggacttttgcggcaagggctaccacctgatgagcttccctcagtctgcccctcacggcgtggtgtttctgcacgtgacatat gtgcccgctcaagagaagaatttcaccaccgctccagccatctgccacgacggcaaagcccactttcctagagaaggcgtgttcgtgtccaac ggcacccattggttcgtgacacagcggaacttctacgagccccagatcatcaccaccgacaacaccttcgtgtctggcaactgcgacgtcgtg atcggcattgtgaacaataccgtgtacgaccctctgcagcccgagctggacagcttcaaagaggaactggacaagtactttaagaaccacac aagccccgacgtggacctgggcgatatcagcggaatcaatgccagcgtcgtgaacatccagaaagagatcgaccggctgaacgaggtggc |
| | | caagaatctgaacgagagcctgatcgacctgcaagaactggggaagtacgagcagtacatcaagtggccctggtacatctggctgggcttta tcgccggactgattgccatcgtgatggtcacaatcatgctgtgttgcatgaccagctgctgtagctgcctgaagggctgttgtagctgtggcagc tgctgcaagttcgacgaggacgattctgagcccgtgctgaagggcgtgaaactgcactacacatgatgactcgagctggtactgcatgcacg caatgctagctgcccctttcccgtcctgggtaccccgagtctcccccgacctcgggtcccaggtatgctcccacctccacctgccccactcaccac ctctgctagttccagacacctcccaagcacgcagcaatgcagctcaaaacgcttagcctagccacacccccacgggaaacagcagtgattaa cctttagcaataaacgaaagtttaactaagctatactaaccccagggttggtcaatttcgtgccagccacaccctggagctagcaaaaaaaa aaaaaaaaaaaaaaaaaaaaaagcatatgactaaaaaaaaaaaaaaaaaaaaaaaaaaaaaaaaaaaaaaaaaaaaaaaaaaaaaaaa aaaaaaaaaaaaaaa |

### Lipid nanoparticles (LNPs)

In one embodiment, nucleic acid such as RNA described herein is administered in the form of lipid nanoparticles (LNPs) (e.g., encapsulated within or associated with an LNP). The LNP may comprise any lipid capable of forming a particle to which the one or more nucleic acid molecules are attached, or in which the one or more nucleic acid molecules are encapsulated.

In one embodiment, LNP comprise a cationic lipid, a neutral lipid, a steroid, a polymer conjugated lipid; and RNA. In one embodiment, the cationic lipid is ALC-0315, the neutral lipid is DSPC, the steroid is cholesterol, and the polymer conjugated lipid is ALC-0159.

In one embodiment, the LNP comprises from 20 to 60 mol percent, 40 to 55 mol percent, from 40 to 50 mol percent, from 41 to 49 mol percent, from 41 to 48 mol percent, from 42 to 48 mol percent, from 43 to 48 mol percent, from 44 to 48 mol percent, from 45 to 48 mol percent, from 46 to 48 mol percent, from 47 to 48 mol percent, or from 47.2 to 47.8 mol percent of the cationic lipid. In one embodiment, the LNP comprises about 47.0, 47.1, 47.2, 47.3, 47.4, 47.5, 47.6, 47.7, 47.8, 47.9 or 48.0 mol percent of the cationic lipid.

In one embodiment, the neutral lipid is present in a concentration ranging from 5 to 25 mol percent, 5 to 15 mol percent, from 7 to 13 mol percent, or from 9 to 11 mol percent. In one embodiment, the neutral lipid is present in a concentration of about 9.5, 10 or 10.5 mol percent.

In one embodiment, the steroid is present in a concentration ranging from 25 to 55 mol percent, 30 to 50 mol percent, from 35 to 45 mol percent or from 38 to 43 mol percent. In one embodiment, the steroid is present in a concentration of about 40, 41, 42, 43, 44, 45 or 46 mol percent.

In some embodiments, an LNP comprises molar ratios of 20-60% ionizable cationic lipid, 5-25% neutral lipid, 25-55% sterol, and 0.5-15% PEG-modified lipid.

The preferred mode of administration is intramuscular administration, more preferably in aqueous cryoprotectant buffer for intramuscular administration. Drug product is a preferably a preservative-free, sterile dispersion of RNA formulated in lipid nanoparticles (LNP) in aqueous cryoprotectant buffer for intramuscular administration.

In different embodiments, drug product comprises the components shown below, e.g., at the proportions or concentrations shown below:

| **Component** | **Function** | **Proportion (mol%)** |
|---|---|---|
| ALC-0315 [1] | Functional lipid | 47.5 |
| ALC-0159 [2] | Functional lipid | 1.8 |
| DSPC [3] | Structural lipid | 10.0 |
| Cholesterol, synthetic | Structural lipid | 40.7 |

| **Component** | **Function** | **Concentration (mg/mL)** |
|---|---|---|
| Drug Substance | Active | 0.5 |
| ALC-0315 [1] | Functional lipid | 7.17 |
| ALC-0159 [2] | Functional lipid | 0.89 |
| DSPC [3] | Structural lipid | 1.56 |
| Cholesterol, synthetic | Structural lipid | 3.1 |
| Sucrose | Cryoprotectant | 102.69 |
| NaCl | Buffer | 6.0 |
| KCl | Buffer | 0.15 |
| Na₂HPO₄ | Buffer | 1.08 |
| KH₂PO₄ | Buffer | 0.18 |
| Water for injection | Solvent/Vehicle | q.s. |
| Drug Substance | Active | 1.0 |
| ALC-0315 [1] | Functional lipid | 13.56 |
| ALC-0159 [2] | Functional lipid | 1.77 |
| DSPC [3] | Structural lipid | 3.11 |
| Cholesterol, synthetic | Structural lipid | 6.20 |
| Sucrose | Cryoprotectant | 102.69 |
| NaCl | Buffer | 6.0 |
| KCl | Buffer | 0.15 |
| Na2HPO4 | Buffer | 1.08 |
| KH2PO4 | Buffer | 0.15 |
| Water for injection | Solvent/Vehicle | q.s. |

| | | |
|---|---|---|
| [1] ALC-0315 = ((4-hydroxybutyl)azanediyl)bis(hexane-6,1-diyl)bis(2-hexyldecanoate) / 6-[N-6-(2-hexyldecanoyloxy)hexyl-N-(4-hydroxybutyl)amino]hexyl 2-hexyldecanoate [2] ALC-0159 = 2-[(polyethylene glycol)-2000]-*N,N*-ditetradecylacetamide / 2-[2-(ω-methoxy (polyethyleneglycol2000) ethoxy]-N,N-ditetradecylacetamide [3] DSPC = 1,2-Distearoyl-*sn*-glycero-3-phosphocholine q.s. = quantum satis (as much as may suffice) | | |

In some embodiments, particles disclosed herein are formulated in a solution comprising 10 mM Tris and 10% sucrose, and optionally having a pH of about 7.4. In some embodiments, particles disclosed herein are formulated in a solution comprising about 103 mg/ml sucrose, about 0.20 mg/ml tromethamine (Tris base), and about 1.32 mg/ml Tris.

In some embodiments, a composition comprises:
(a) about 0.1 mg/mL RNA comprising an open reading frame encoding a polypeptide that comprises a SARS-CoV-2 protein or an immunogenic fragment or variant thereof,
(b) about 1.43 mg/ml ALC-0315,
(c) about 0.18 mg/ml ALC-0159,
(d) about 0.31 mg/ml DSPC,
(e) about 0.62 mg/ml cholesterol,
(f) about 103 mg/ml sucrose,
(g) about 0.20 mg/ml tromethamine (Tris base),
(h) about 1.32 mg/ml Tris (hydroxymethyl) aminomethane hydrochloride (Tris HCl), and
(i) q.s. water.

In one embodiment, the ratio of RNA (e.g., mRNA) to total lipid (N/P) is between 6.0 and 6.5 such as about 6.0 or about 6.3.

In some embodiments, compositions provided herein are formulated as a multi-dose formulation, optionally in a vial.

### Methods of Administering

Also described in the present disclosure, among other things, are methods that comprise administering a composition described herein. In some embodiments, methods described herein induce an immune response in a subject (e.g., an immune response against coronavirus).

In some embodiments, an amount of the RNA described herein of at least 0.25 µg, at least 0.5 µg, at least 1 µg, at least 2 µg, at least 3 µg, at least 4 µg, at least 5 µg, at least 10 µg, at least 15 µg, at least 20 µg, at least 25 µg, at least 30 µg, at least 40 µg, at least 50 µg, or at least 60 µg may be administered per dose (e.g., in a given dose). In some embodiments, an amount of the RNA described herein of at least 3 ug may be administered in at least one of given doses. In some embodiments, an amount of the RNA described herein of at least 10 ug may be administered in at least one of given doses. In some embodiments, an amount of the RNA described herein of at least 15 ug may be administered in at least one of given doses. In some embodiments, an amount of the RNA described herein of at least 20 ug may be administered in at least one of given doses. In some embodiments, an amount of the RNA described herein of at least 25 ug may be administered in at least one of given doses. In some embodiments, an amount of the RNA described herein of at least 30 ug may be administered in at least one of given doses. In some embodiments, combinations of aforementioned amounts may be administered in a regimen comprising two or more doses (e.g., a prior dose and a subsequent dose can be of different amounts as described herein). In some embodiments, combinations of aforementioned amounts may be administered in a primary regimen and a booster regimen (e.g., different doses can be given in a primary regimen and a booster regimen).

In some embodiments, a composition comprising an RNA described herein is administered as a first dose and/or as part of a priming vaccination regimen to a subject (e.g., a vaccine-naïve subject is administered (i) two doses of such a composition, approximately 21 days apart, or (ii) three doses of such a composition, where the first and the second doses are administered approximately 21 days apart and the second and the third dose are administered about 28 days apart).

In some embodiments, a composition that comprises an RNA described herein is administered to a subject who has previously been exposed to SARS-CoV-2 (e.g., a subject who has previously received at least one dose (e.g., a complete dosing regimen) of a SARS-CoV-2 vaccine and/or previously been infected one or more times with SARS-CoV-2). In some embodiments, a composition comprising an RNA described herein is administered as a booster dose. In some embodiments, a composition comprising an RNA described herein is administered as a booster dose to a subject who has previously received one or more doses (e.g., a complete primary dosing regimen and/or one or more booster doses) of a vaccine that delivers a SARS-CoV-2 S protein of a Wuhan strain (e.g., a composition comprising an RNA that comprises SEQ ID NO: 20, a commercially available vaccine (e.g., a commercially available vaccine described herein, (e.g., BNT162b2)) or any combination thereof).

Commercially available SARS-CoV-2 vaccines are known in the art, and include, e.g., an mRNA-1273 vaccine, an Ad26.CoV2.S vaccine, a ChAdxOx1 vaccine, an NVX-CoV2373 vaccine, a CvnCoV vaccine, a GAM-COVID0Vac vaccine, a CoronaVac vaccine, a BBIBP-CorV vaccine, an Ad5-nCoV vaccine, a zf2001 vaccine, a SCB-2019 vaccine, or other approved RNA (e.g., mRNA) or adenovector vaccines, etc.

In some embodiments, an RNA described herein is administered to a subject who has previously been administered at least two doses of BNT162b2 (e.g., two doses of BNT162b2 administered about 21 days apart). In some embodiments, an RNA described herein is administered to a subject who has previously been administered at least three doses of BNT162b2 embodiment, at least three doses are administered. In some embodiments, such third dose is administered a period of time after the second dose that is comparable to (e.g., the same as) the period of time between the first and second doses. For example, in some embodiments, a third dose may be administered about 21 days following administration of the second dose. In some embodiments, a third dose is administered after a longer period of time relative to the second dose than the second dose was relative to the first dose. In some embodiments, a three-dose regimen is administered to an immunocompromised patient, e.g., a cancer patient, an HIV patient, a patient who has received and/or is receiving immunosuppressant therapy (e.g., an organ transplant patient). In some embodiments, the length of time between the second and third dose (e.g., a second and third dose administered to an immunocompromised patient) is at least about 21 days (e.g., at least about 28 days).

In some embodiments, an RNA described herein is administered to a subject who has previously been administered a vaccine that delivers an antigen of a SARS-CoV-2 variant (e.g., an Omicron BA.4/5 variant). For example, in some embodiments, an RNA described herein is administered to a subject previously administered one or more doses of a SARS-CoV-2 vaccine that delivers a SARS-CoV-2 S protein of a Wuhan strain (e.g., BNT162b2), and one or more booster doses of a variant adapted vaccine (e.g., one or more doses of a bivalent vaccine that delivers a SARS-CoV-2 S protein of a Wuhan strain and a SARS-CoV-2 S protein of an Omicron BA.4/5 strain).

BNT162b2 (which comprises an RNA comprising SEQ ID NO: 20) is an mRNA vaccine for prevention of COVID-19 and demonstrated an efficacy of 95% or more at preventing COVID-19. The vaccine comprises a 5'capped mRNA encoding for the full-length SARS-CoV-2 spike glycoprotein (S) encapsulated in lipid nanoparticles (LNPs). The finished product is presented as a concentrate for dispersion for injection containing BNT162b2 as active substance. Other ingredients include: ALC-0315 (4-hydroxybutyl)azanediyl)bis(hexane-6,1-diyl)bis(2-hexyldecanoate), ALC-0159 (2-[(polyethylene glycol)-2000]-N,N-ditetradecylacetamide), 1,2-Distearoyl-sn-glycero-3-phosphocholine (DSPC), cholesterol, and, in some embodiments, potassium chloride, potassium dihydrogen phosphate, sodium chloride, disodium phosphate dihydrate, sucrose and water for injection.

In some embodiments, a composition comprising an RNA described herein is administered to a subject previously administered a priming dosing regimen of a composition (e.g., an RNA composition) that delivers a SARS-CoV-2 S protein of a Wuhan strain (e.g., a subject previously administered (i) two doses of an RNA vaccine that encodes a SARS-CoV-2 S protein of a Wuhan strain, where the first and the second doses were administered about 21 days apart, or (ii) three doses of an RNA vaccine that encodes a SARS-CoV-2 S protein of a Wuhan strain, where the first and the second dose were administered about 21 days apart and the third dose was administered about 28 days after the second dose).

In some embodiments, a composition comprising an RNA described herein was administered as a further booster dose to a subject previously administered a priming dosing regimen and one or more booster doses of a composition (e.g., an RNA composition) that delivers a SARS-CoV-2 S protein of a Wuhan strain (e.g., a subject previously administered (i) three doses of an RNA vaccine that encodes a SARS-CoV-2 S protein of a Wuhan strain, where the first and the second doses were administered about 21 days apart, and the third dose was administered at least about 2 months after the second dose, (ii) four doses of an RNA vaccine that encodes a SARS-CoV-2 S protein of a Wuhan strain, where the first and the second dose were administered about 21 days apart, the third dose was administered about 28 days after the second dose, and the fourth dose was administered at least about three months after the second dose, or (iii) four doses of an RNA vaccine that encodes a SARS-CoV-2 S protein of a Wuhan strain, where the first and the second dose were administered about 21 days apart, the third dose was administered at least about 2 months after the second dose, and the fourth dose was administered at least about two months after the second dose).

In some embodiments, a composition comprising an RNA described herein is administered to a subject previously administered one or more doses of a bivalent composition (e.g., an RNA composition) that delivers a SARS-CoV-2 S protein of a Wuhan strain and a SARS-CoV-2 S protein of an Omicron BA.4/5 variant. In some embodiments, a composition comprising an RNA described herein is administered as a booster dose to a subject previously administered a priming dosing regimen of a bivalent composition (e.g., an RNA composition) that delivers a SARS-CoV-2 S protein of a Wuhan strain and a SARS-CoV-2 S protein of an Omicron BA.4/5 variant (e.g., a subject previously administered (i) two doses of a bivalent RNA vaccine, where the first and the second doses were administered about 21 days apart, or (ii) three doses of a bivalent RNA vaccine, where the first and the second dose were administered about 21 days apart and the third dose was administered about 28 days after the second dose). In some embodiments, a composition comprising an RNA described herein is administered as a further booster dose to a subject previously administered a priming dosing regimen and one or more booster doses of a bivalent composition (e.g., an RNA composition) that delivers a SARS-CoV-2 S protein of a Wuhan strain and a SARS-CoV-2 S protein of an Omicron BA.4/5 variant (e.g., a subject previously administered (i) three doses of a bivalent RNA vaccine, where the first and the second doses were administered about 21 days apart, and the third dose was administered at least about 2 months after the second dose, (ii) four doses of a bivalent RNA vaccine, where the first and the second dose were administered about 21 days apart, the third dose was administered about 28 days after the second dose, and the fourth dose was administered at least about three months after the second dose, or (iii) four doses of a bivalent RNA vaccine, where the first and the second dose were administered about 21 days apart, the third dose was administered at least about 2 months after the second dose, and the fourth dose was administered at least about two months after the second dose).

In some embodiments, a composition comprising an RNA described herein is administered to a subject previously administered (i) one or more doses of a composition that delivers a SARS-CoV-2 S protein of a Wuhan strain and (ii) one or more doses of a bivalent composition (e.g., an RNA composition) that delivers a SARS-CoV-2 S protein of a Wuhan strain and a SARS-CoV-2 S protein of an Omicron BA.4/5 variant.

In some embodiments, a composition comprising an RNA described herein is administered to a subject previously administered:
(ii) two doses (administered about 21 days apart) of an RNA vaccine that delivers a SARS-CoV-2 S protein of a Wuhan strain, and a dose of a bivalent composition comprising a first RNA encoding a SARS-CoV-2 S protein of a Wuhan strain and a second RNA encoding a SARS-CoV-2 S protein of an Omicron BA.4/5 variant, where the bivalent composition was administered at least about two months after the most recent dose of a composition that delivers a SARS-CoV-2 S protein of a Wuhan strain;
(iii) three doses of an RNA vaccine that delivers a SARS-CoV-2 S protein of a Wuhan strain (where the first and the second dose were administered about 21 days apart and the third dose was administered about 28 days after the second dose) and at least one dose of a bivalent vaccine comprising a first RNA encoding a SARS-CoV-2 S protein of a Wuhan strain and a second RNA encoding a SARS-CoV-2 S protein of an Omicron BA.4/5 variant, where the bivalent composition was administered at least about 2 months after the most recent dose of a composition that delivers a SARS-CoV-2 S protein of a Wuhan strain;
(iv) three doses of an RNA vaccine that delivers a SARS-CoV-2 S protein of a Wuhan strain (where the first and the second dose were administered about 21 days apart and the third dose was administered at least about 2 months after the second dose) and at least one dose of a bivalent vaccine comprising a first RNA encoding a SARS-CoV-2 S protein of a Wuhan strain and a second RNA encoding a SARS-CoV-2 S protein of an Omicron BA.4/5 variant, where the bivalent composition was administered at least about 2 months after the most recent dose of a composition that delivers a SARS-CoV-2 S protein of a Wuhan strain.
(v) four doses of an RNA vaccine that delivers a SARS-CoV-2 S protein of a Wuhan strain (where the first and the second dose were administered about 21 days apart, the third dose was administered about 28 days after the second dose, and the fourth dose was administered at least about 2 months after the third dose) and at least one dose of a bivalent vaccine comprising a first RNA encoding a SARS-CoV-2 S protein of a Wuhan strain and a second RNA encoding a SARS-CoV-2 S protein of an Omicron BA.4/5 variant, where the bivalent composition was administered at least about 2 months after the most recent dose of a composition that delivers a SARS-CoV-2 S protein of a Wuhan strain; or four doses of an RNA vaccine that delivers a SARS-CoV-2 S protein of a Wuhan strain (where the first and the second dose were administered about 21 days apart, the third dose was administered at least about 2 months after the second dose, and the fourth dose was administered at least about 4 months after the third dose) and at least one dose of a bivalent vaccine comprising a first RNA encoding a SARS-CoV-2 S protein of a Wuhan strain and a second RNA encoding a SARS-CoV-2 S protein of an Omicron BA.4/5 variant, where the bivalent composition was administered at least about 2 months after the most recent dose of a composition that delivers a SARS-CoV-2 S protein of a Wuhan strain.

In some embodiments, a dose (e.g., a dose administered as part of a primary dosing regimen or a booster regimen) comprises about 30 µg of an RNA described herein. In some embodiments, a dose comprising about 30 µg of RNA described herein is administered to a subject who is 12 years or older.

In some embodiments, a dose (e.g., a dose administered as part of a primary dosing regimen or a booster regimen) comprises about 10 µg of an RNA described herein. In some embodiments, a dose comprising about 10 µg of RNA described herein is administered to a subject who is 5 years to less than 12 years old.

In some embodiments, a dose (e.g., a dose administered as part of a primary dosing regimen or a booster regimen) comprises about 3 µg of an RNA described herein. In some embodiments, a dose comprising about 3 µg of RNA described herein is administered to a subject who is 6 months to less than 5 years old.

In some embodiments, a composition described herein is administered in a volume of between about 200 µl and about 300 µl (e.g., about 200 µl or about 300 µl).

In some embodiments, RNA in a pharmaceutical RNA preparation is diluted prior to administration (e.g., diluted to a concentration of about 0.05 mg/ml). In some embodiments, administration volumes are between about 200 µl and about 300 µl. In some embodiments, RNA in a pharmaceutical RNA preparation is formulated in about 10 mM Tris buffer, and about 10% sucrose.

In some embodiments, an RNA (e.g., mRNA) composition disclosed herein may be administered in conjunction with a vaccine targeting a different infectious agent. In some embodiments, the different infectious agent is one that increases the likelihood of a subject experiencing deleterious symptoms when coinfected with SARS-CoV-2 and the infectious agent. In some embodiments, the infectious agent is one that increases the infectivity of SARS-CoV-2 when a subject is coinfected with SARS-CoV-2 and the infectious agent. In some embodiments, at least one RNA (e.g., mRNA) composition described herein may be administered in combination with a vaccine that targets influenza. In some embodiments, at least two or more different drug products/formulations may comprise at least one RNA (e.g., mRNA) composition described herein and a vaccine targeting a different infectious agent (e.g., an influenza vaccine). In some embodiments, different drug products/ formulations are separately administered. In some embodiments, such different drug product/formulations are separately adminsitered at the same time (e.g., at the same vaccination session) at different sites of a subject (e.g., at different arms of the subject).

In one embodiment, the vaccination regimen comprises a first vaccination using at least two doses of the RNA described herein, e.g., two doses of the RNA described herein (wherein the second dose may be administered about 21 days following administration of the first dose), and a second vaccination using a single dose or multiple doses, e.g., two doses, of the RNA described herein. In various embodiments, the second vaccination is administered at least about 2 months after a previous dose (e.g., 3 to 24 months, 6 to 18 months, 6 to 12 months, or 5 to 7 months after administration of a previous vaccine, e.g., after an initial two-dose regimen or a booster dose). The amount of RNA used in each dose of the second vaccination may be equal or different to the amount of RNA used in each dose of the first vaccination. In one embodiment, the amount of RNA used in each dose of the second vaccination is equal to the amount of RNA used in each dose of the first vaccination. In one embodiment, the amount of RNA used in each dose of the second vaccination and the amount of RNA used in each dose of the first vaccination is about 30 µg per dose. In one embodiment, the same RNA as used for the first vaccination is used for the second vaccination.

In some embodiments, an RNA composition described herein is co-administered with one or more vaccines against a non-SARS-CoV-2 disease. In some embodiments, an RNA composition described herein is co-administered with one or more vaccines against a non-SARS-COV-2 viral disease. In some embodiments, an RNA composition described herein is co-administered with one or more vaccines against a non-SARS-CoV-2 respiratory disease. In some embodiments, the non-SARS-CoV-2 respiratory disease is a non-SARS-CoV-2 Coronavirus, an Influenza virus, a Pneumoviridae virus, or a Paramyxoviridae virus. In some embodiments, the Pneumoviridae virus is a Respiratory syncytial virus or a Metapneumovirus. In some embodiments, the Metapneumovirus is a human metapneumovirus (hMPV). In some embodiments, the Paramyxoviridae virus is a Parainfluenza virus or a Henipavirus. In some embodiments the parainfluenzavirus is PIV3. In some embodiments, the non-SARS-CoV-2 coronavirus is a betacoronavirus (e.g., SARS-CoV-1). In come embodiments the non-SARS-CoV-2 coronavirus is a Merbecovirus (e.g., a MERS-CoV virus).

In some embodiments, an RNA composition described herein is co-administered with an RSV vaccine (e.g., an RSV A or RSV B vaccine). In some embodiments, the RSV vaccine comprises an RSV fusion protein (F), an RSV attachment protein (G), an RSV small hydrophobic protein (SH), an RSV matrix protein (M), an RSV nucleoprotein (N), an RSV M2-1 protein, an RSV Large polymerase (L), and/or an RSV phosphoprotein (P), or an immunogenic fragment of immunogenic variant thereof, or a nucleic acid (e.g., RNA), encoding any one of the same.

Numerous RSV vaccines are known in the art, any one of which can be co-administered with an RNA composition described herein. See, for example, the list of RSV vaccines provided on the website of PATH, a global health organization (see https://www.patch.org/resources/rsv-vaccine-and-mab-snapshot/), as well as in Mazur, Natalie I., et al, "The respiratory syncytial virus vaccine landscape: lessons from the graveyard and promising candidates," The Lancet Infectious Diseases 18.10 (2018): e295-e311, the contents of each of which is incorporated by reference herein. In some embodiments, an RNA composition described herein is co-administered with an RSV vaccine that has been previously published on (e.g., an RSV vaccine described on the PATH website page linked to above, or in Mazur et al.). In some embodiments, an RNA composition described herein is co-administered with a live-attenuated or chimeric vaccine (e.g., rBCG-N-hRSV (developed by Ponteificia Uinersidad Catolica de Chile), RSV D46 cp ΔM202 (developed by Sanofi Pasteur/LID/NIAD/NIH), RSV LID ΔM2-2 1030s (developed by Sanofi Pasteur/LID/NIAD/NIH), RSV ΔNS2 Δ1313/I1314L (developed by Sanofi Pasteur/LID/NIAD/NIH), RSV D46 ΔNS2 N ΔM2-2 HindIII (developed by Sanofi Pasteur/LID/NIAD/NIH) or RSV LID ΔM2-2 1030s (developed by Sanofi Pasteur/LID/NIAD/NIH), MV-012-968 (developed by Meissa Vaccines), SP0125 (developed by Sanofi), blb201 (developed by Blue lake), CodaVax^{™}-RSV (developed by Cadagenix), RSVDeltaG (developed by Intravacc), or SeVRSV (developed by SIHPL and St. Jude hospital), a particle based vaccine (e.g., RSV F nanoparticle (developed by Novavax) or SynGEM (developed by Mucosis), Icosavzx (developed by IVX-121), or V-306 (developed by Virometix)), a subunit vaccine (e.g., GSK RSV F (developed by GSK), Arexvy (developed by GSK), DPX-RSV (developed by Dalousie Univeristy, Immunovaccine, and VIB), RSV F DS-Cav1 (developed by NIH/NIAID/VRC), MEDI-7510 (developed by MedImmune), RSVpreF (developed by Pfizer), ADV110 (developed by Advaccine), VN-0200 (developed by Daiichi Sankyo, Inc.)), a vector vaccine (e.g., MVA-BN RSV (developed by Banarian Nordic), VXA-RSVf oral (developed by Vaxart), Ad26.RSV.pref (developed by Janssen), ChAd155-RSV (developed by GSK) Immunovaccine, DPX-RSV (developed by VIB), or DS-Cav1 (developed by NIH/NIAID/VRC) or a nucleic acid vaccine (e.g., an mRNA vaccine being developed by CureVac (currently unnamed) or mRNA-1345 (developed by Moderna), or SP0274 (developed by Sanofi)).

In some embodiments, an RNA composition described herein is co-administered with an influenza vaccine. In some embodiments, the influenza vaccine is an alpha-influenza virus, a beta-influenza virus, a gamma-influenza virus or a delta-influenza virus vaccine. In some embodiments the vaccine is an Influenza A virus, an Influenza B virus, an Influenza C virus, or an Influenza D virus vaccine. In some embodiments, the influenza A virus vaccine comprises a hemagglutinin selected from H1, H2, H3, H4, H5, H6, H7, H8, H9, H10, H11, H12, H13, H14, H15, H16, H17, and H18, or an immunogenic fragment or variant of the same, or a nucleic acid (e.g., RNA) encoding any one of the same. In some embodiments the influenza A vaccine comprises or encodes a neuraminidase (NA) selected from N1, N2, N3, N4, N5, N6, N7, N8, N9, N10, and N11, or an immunogenic fragment or variant of the same, or a nucleic acid (e.g., RNA) encoding any one of the same. In some embodiments, the influenza vaccine comprises at least one Influenza virus hemagglutinin (HA), neuraminidase (NA), nucleoprotein (NP), matrix protein 1 (M1), matrix protein 2 (M2), non-structural protein 1 (NS1 ), non-structural protein 2 (NS2), nuclear export protein (NEP), polymerase acidic protein (PA), polymerase basic protein PB1, PB1-F2, and/or polymerase basic protein 2 (PB2), or an immunogenic fragment or variant thereof, or a nucleic acid (e.g., RNA) encoding any of one of the same.

In some embodiments, an RNA composition described herein can be co-administered with a commercially approved influenza vaccine. In some embodiments, an RNA composition described herein can be co-administered with an inactivated influenza virus (e.g., Fluzone^{®}, Fluzone high-dose quadrivalent^{®}, Fluzone quadrivalent^{®}, Fluzone intradermal quardivalent^{®}, Fluzone quadrivalent southern hemisphere^{®}, Fluad^{®}, Fluad quadrivalent^{®}, Afluria Quardivalent^{®}, Fluarix Quadrivalent^{®}, FluLaval Quadrivalent^{®}, or Flucelvax Quadrivalent^{®}), a recombinant influenza vaccine (e.g., Flublok quadrivalent^{®}), a live attenuated influenza vaccine (e.g., FluMist Quadrivalent^{®}), a non-adjuvanted influenza vaccine, an adjuvanted influenza vaccine, or a subunit or split vaccine.

In some embodiments, an RNA composition described herein is co-administered with an influenza vaccine and/or an RSV vaccine.

In one embodiment, a composition or medical preparation is a pharmaceutical composition.

In one embodiment, a composition or medical preparation is a vaccine.

### Examples

### Example 1: Further Studies of Variant-Adapted Bi- and Trivalent Vaccines in Vaccine-Experienced Mice

The present Example describes a study to characterize immune responses induced by certain variant-adapted vaccines in vaccine-experienced subjects (mice in the present Example). In particular, the present Example describes an experiment to characterize immune responses induced in subjects previously administered (i) at least one dose of a composition that delivers a SARS-CoV-2 S protein of a Wuhan strain, and (ii) at least one dose of a bivalent composition comprising a first RNA that encodes a SARS-COV-2 S protein of a Wuhan strain and a second RNA that encodes a SARS-CoV-2 S protein of a BA.4/5 Omicron variant.

Two sets of experimental dosing regimens are summarized in Figs. 1 and 2.

In a first set of experiments (summarized in Fig. 1), BALB/C mice are administered a first dose of an RNA vaccine encoding a SARS-CoV-2 S protein of a Wuhan variant (BNT162b2), and, 21 days later, a second dose of a bivalent vaccine comprising a first RNA encoding a SARS-CoV-2 S protein of a Wuhan strain and a second RNA encoding SARS-COV-2 S protein of a BA.4/5 Omicron variant. About 10 weeks after administered the second dose, mice are then administered one of the following compositions (comprising RNAs encoding SARS-CoV-2 S proteins from the listed strains/variants):
- Wuhan + BA.4/5
- XBB.1.5
- BA.2.75.2
- BQ.1.1
- Wuhan + BA.4/5 + XBB.1.5
- Wuhan + BA.4/5 + BA.2.75.2
- BA.4/5 + XBB.1.5
- BA.4/5 + BA.2.75.2
- BA.4/5 + BQ.1.1

About 28 days after the third dose, mice are sacrificed and final neutralization titers are collected.

In a related experiment, neutralization titers are determined in mice administered two doses of a composition that delivers a Wuhan S protein, a third dose of a bivalent vaccine, and a fourth dose of one of the vaccines listed below (comprising RNAs encoding SARS-CoV-2 S proteins from the listed variants). The design of this experiment is summarized in Fig. 2.
- XBB.1.5
- BA.2.75.2
- BQ.1.1
- BA.4/5 + XBB.1.5
- BA.4/5 + BA.2.75.2
- BA.4/5 + BQ.1.1

### Example 2: Further Studies Investigating Variant-Adapted Vaccines Administered to Vaccine-Naive Mice

The present Example provides data characterizing certain monovalent and bivalent variant-adapted vaccines in vaccine naive mice. The particular vaccines tested are shown in Fig. 3. As shown in the Figure, vaccines were administered vaccines on days 0 and 21 (the same vaccine composition and dose was administered on each day; dose amounts indicated in Fig. 3), and sera samples were collected on day 35 (14 days after the second dose of a vaccine).

Pseudovirus neutralization titers from sera samples collected on day 35 are shown in Fig. 4. As shown in the figure, each of the tested compositions can produce strong neutralization titers against the corresponding strain or variant. In particular, a monovalent composition comprising an RNA encoding an S protein of an XBB.1.5 Omicron variant was shown to induce high neutralization titers against XBB.1.5 when administered as a primary series. A bivalent composition comprising an RNA encoding an S protein of an XBB.1.5 Omicron variant and an RNA encoding an S protein of an Omicron BA.4/5 variant was also shown to induce high neutralization titers against XBB.1.5 when administered as a primary series, although titers were lower than those induced by an XBB.1.5 monovalent vaccine.

Each of the compositions comprising an RNA encoding an S protein of a SARS-CoV-2 variant (i.e., WT + BA.4/5, BA.4/5, XBB.1.5, and BA.4/5 + XBB.1.5) was also found to induce broad cross-neutralization of other variants of concern. In particular, the BA.4/5 + XBB.1.5 bivalent vaccine was found to induce high neutralization titers both against the XBB.1.5 variant as well as other strains. Neutralization titers against XBB.1.16 are expected to be similar to those induced against XBB.1.5, as neutralization data shows that the XBB.1.16 variant possesses no escape advantage relative to XBB.1.5 (see, e.g., Fig. 6). Similarly, neutralization titers for XBB.1.9.1/1.9.2 are expected to match those for XBB.1.5, as XBB.1.9.1/1.9.2 does not comprise any additional S protein mutations relative to XBB.1.5.

A similar experiment (summarized in the schematic at the top of Fig. 7) was also performed in which vaccine-naive mice were administered two doses of (i) a bivalent composition comprising RNA encoding a SARS-CoV-2 S protein of a Wuhan strain and RNA encoding a SARS-CoV-2 S protein of an Omicron BA.4/5 variant, (ii) a monovalent composition comprising RNA encoding a SARS-CoV-2 S protein of an XBB.1.5 variant, or (iii) a bivalent composition comprising RNA encoding a SARS-CoV-2 S protein of an Omicron BA.4/5 variant and RNA encoding a SARS-CoV-2 S protein of an XBB.1.5 variant. The two doses of RNA were administered 21 days apart, and neutralization titers were collected 4 weeks after administering the second dose. As shown in Fig. 7, an XBB.1.5-adapted monovalent vaccine (e.g., as described herein) induced the highest neutralization titers against each of an XBB.1.5 variant, an XBB.1.16 variant, and an XBB.2.3 variant as compared to the other compositions tested.

### Example 3: Further Studies Investigating Variant-Adapted Vaccines Administered to Vaccine-Experienced Mice

The present Example provides exemplary immune response data generated using certain monovalent and bivalent variant-adapted vaccines in vaccine-experienced mice. In particular, the present Example provides data demonstrating that vaccines (e.g., monovalent or bivalent vaccines) comprising an RNA encoding an XBB.1.5 S protein can induce strong neutralization titers against an XBB.1.5 Omicron variant in previously vaccinated subjects (e.g., subjects previously administered RNA encoding a SARS-CoV-2 S protein of a Wuhan strain and RNA encoding a SARS-CoV-2 S protein of an Omicron BA.4/5 variant, as in the present Example).

The particular vaccines tested are shown in Fig. 5. As shown in the Figure, mice in all groups were administered the following vaccines (listed in order of administration): (1) a first dose of a vaccine comprising RNA encoding a SARS-CoV-2 S protein of a Wuhan strain (BNT162b2 in the present example), (2) a second dose of a vaccine comprising an RNA encoding a SARS-CoV-2 S protein of a Wuhan strain (again, BNT162b2 in the present example; second dose administered about 21 days after the first dose), (3) a third dose of a bivalent vaccine comprising a first RNA encoding a SARS-CoV-2 S protein of a Wuhan strain and a second RNA encoding a SARS-CoV-2 S protein of an Omicron BA.4/5 variant (administered about 84 days after the second dose), and (4) a fourth dose of one of the monovalent or bivalent vaccines listed in the Figure (administered about 29 days after the third dose). Sera samples were collected 134 days after administration of the first dose of vaccine (immediately before administering the 4^{th} dose), and 160 days after administering the 1^{st} dose. Mice were administered 0.5 µg of each vaccine (for monovalent vaccines: 0.5 µg of an RNA, for bivalent vaccines: 0.25 µg of each RNA). On day 160, lymph nodes and spleen samples were also collected for T cell and B cell analysis.

Pseudovirus neutralization titers are shown in Fig. 6. As shown in the figure, vaccines comprising an RNA encoding an XBB.1.5 S protein were found to induce a strong neutralization response when administered as either a monovalent or bivalent (e.g., BA.4/5 + XBB.1.5) vaccine. In particular, the data demonstrates that such vaccines are especially effective when administered as a booster dose (e.g., to subjects previously administered one or more vaccines that deliver a SARS-CoV-2 S protein of a Wuhan strain and one or more bivalent vaccines (delivering a SARS-CoV-2 S protein of a Wuhan strain and an Omicron BA.4/5 variant), as tested in the present Example). In the present experiment, neutralization titers induced by an XBB.1.5 monovalent vaccine (in particular, neutralization titers against an XBB.1.5, XBB.1.16, and XBB.2.3-adapted pseudovirus) were found to be higher than those induced by a bivalent vaccine (BA.4/5 + XBB.1.5). Other studies confirmed that monovalent or bivalent vaccine comprising an RNA encoding an XBB.1.5 S protein can induce a strong immune response against XBB variants (in particular, when administered as a booster), and further showed that, while a bivalent vaccine can induce higher neutralization titers at early time points, neutralization titers induced by a bivalent vaccine decreased faster than those induced by a monovalent vaccine, such that, 4 weeks after a booster dose, neutralization titers induced by a monovalent vaccine were higher than those induced by a bivalent vaccine (data not shown). As noted in previous Example 2, neutralization titers for XBB.1.9.1/1.9.2 are expected to match those induced for XBB.1.5, as XBB.1.9.1/1.9.2 does not comprise any additional S protein mutations relative to XBB.1.5.

A similar experiment was also performed to test the efficacy of an XBB.1.16-adapted vaccine (schematic summarizing the experimental protocol used is shown in Fig. 8). In short, mice were divided into groups of 10, and administered two doses (21 days apart) of an RNA encoding a SARS-CoV-2 S protein of a Wuhan strain (BNT162b2 in the present example), followed by a third dose (administered 14 days after the second dose) of a candidate vaccine encoding (i) a bivalent composition comprising a first RNA encoding a SARS-CoV-2 S protein of a Wuhan strain and a second RNA encoding a SARS-CoV-2 S protein of an Omicron BA.4/5 variant, (ii) a monovalent composition comprising an RNA encoding a SARS-CoV-2 S protein of an XBB.1.5 variant, (iii) a monovalent composition comprising an RNA encoding a SARS-CoV-2 S protein of an XBB.1.16 variant. 14 days after administering a third dose of vaccine, mice were bled and neutralization titers collected (e.g., using a pseudovirus neutralization assay described in one of the previous examples).

As shown in Fig. 8, a monovalent composition comprising an RNA encoding a SARS-CoV-2 S protein of an XBB.1.5 variant (e.g., an RNA described herein) induced the highest neutralization titers against each of the XBB.1.5, XBB.1.16, and XBB.2.3 variants. Surprisingly, an XBB.1.5 monovalent composition induced higher titers against an XBB.1.16 variant than a monovalent comprising an RNA encoding a SARS-CoV-2 S protein of an XBB.1.16 variant.

### Example 4: Description of Sequences Provided in Sequence Listing

Table 2, below, provides a short description of sequences provided in the sequence listing submitted herewith.

| SEQ ID NO | Description |
|---|---|
| 1 | Amino acid sequence of a full length SARS-CoV-2 S protein of a Wuhan strain |
| 2 | Exemplary RNA sequence encoding a full length SARS-CoV-2 S protein of a Wuhan strain |
| 3 | Exemplary amino acid sequence of a receptor binding domain (RBD) of a Wuhan SARS-CoV-2 S protein |
| 4 | Exemplary RNA sequence encoding an RBD of a Wuhan SARS-CoV-2 S protein |
| 5 | Exemplary amino acid sequence of an RBD of a Wuhan SARS-CoV-2 S protein fused to a trimerization domain (fibritin) |
| 6 | Exemplary RNA sequence encoding an RBD of a Wuhan SARS-CoV-2 strain fused to a trimerization domain (fibritin) |
| 7 | Exemplary amino acid sequence of a full length Wuhan SARS-CoV-2 S protein comprising prefusion stablizing mutations (proline substitutions at positions 986 and 987) |
| 8 | Exemplary RNA sequence encoding a full length Wuhan SARS-CoV-2 S protein comprising prefusion stablizing mutations (proline substitutions at positions 986 and 987) |
| 9 | Exemplary RNA sequence encoding a full length Wuhan SARS-CoV-2 S protein comprising prefusion stablizing mutations (proline substitutions at positions 986 and 987) |
| 10 | Exemplary amino acid sequence of a "foldon" trimerization domain |
| 11 | Exemplary RNA sequence encoding a "foldon" trimerization domain |
| 12 | Exemplary 5' UTR sequence |
| 13 | Exemplary 3' UTR sequence |
| 14 | Exemplary polyA sequence |
| 15 | Exemplary RNA sequence encoding a full length SARS-CoV-2 S protein of a Wuhan strain comprising prefusion stablizing mutations (proline substitutions at positions 986 and 987) |
| 16 | Exemplary RNA sequence encoding a full length SARS-CoV-2 S protein of a Wuhan strain comprising prefusion stablizing mutations (proline substitutions at positions 986 and 987) |
| 17 | Exemplary RNA sequence encoding an RBD of a Wuhan SARS-CoV-2 S protein fused to a trimerization domain (fibritin) |
| 18 | Exemplary amino acid sequence of an RBD of a Wuhan SARS-CoV-2 S protein fused to a trimerization domain (fibritin) |
| 19 | Exemplary RNA sequence comprising a sequence encoding a full length, prefusion stabilized SARS-CoV-2 S protein of a Wuhan strain, along with 5' UTR, 3'UTR, and polyA sequences |

| | |
|---|---|
| 20 | Exemplary RNA sequence comprising a sequence encoding a full length, prefusion stabilized SARS-CoV-2 S protein of a Wuhan strain, along with 5' UTR, 3'UTR, and polyA sequences |
| 21 | Exemplary RNA sequence comprising a sequence encoding an RBD of a Wuhan SARS-CoV-2 S protein fused to a trimerization domain (fibritin), along with 5' UTR, 3'UTR, and polyA sequences |
| 22 | Exemplary amino acid sequence of alphavirus nsp1-4 |
| 23 | Exemplary amino acid sequence of an alphavirus non-structural protein |
| 24 | Exemplary saRNA sequence encoding a full length SARS-CoV-2 S protein of a Wuhan strain comprising prefusion stablizing mutations (proline substitutions at positions 986 and 987) |
| 25 | Exemplary saRNA sequence encoding a full length SARS-CoV-2 S protein of a Wuhan strain comprising prefusion stablizing mutations (proline substitutions at positions 986 and 987) |
| 26 | Exemplary saRNA sequence encoding an RBD of a Wuhan strain fused to a trimerization domain (fibritin) |
| 27 | Exemplary saRNA sequence encoding an RBD of a Wuhan SARS-CoV-2 S protein fused to a trimerization domain (fibritin) and a transmembrane domain |
| 28 | Exemplary amino acid sequence of an RBD of a Wuhan SARS-CoV-2 S protein fused to a trimerization domain (fibritin) and a transmembrane domain |
| 29 | Exemplary amino acid sequence of an RBD of a Wuhan SARS-CoV-2 S protein fused to a trimerization domain (fibritin) and a transmembrane domain |
| 30 | Exemplary RNA sequence encoding an RBD of a Wuhan SARS-CoV-2 S protein fused to a trimerization domain (fibritin) and a transmembrane domain |
| 31 | Exemplary amino acid sequence of an RBD of a Wuhan SARS-CoV-2 S protein fused to a trimerization domain (fibritin) and a transmembrane domain |
| 32 | Exemplary RNA sequence encoding an RBD of a Wuhan SARS-CoV-2 S protein fused to a trimerization domain (fibritin) and a transmembrane domain |
| 33 | Exemplary GS linker |
| 34 | Exemplary GS linker |
| 35-42, 142-145 | Exemplary T cell epitopes recognized by vaccine-induced T cells (e.g., CD8+ T cell) and presented by an MHC class I allele present in at least 50% of subjects in a population |
| 43-48, 138-141, 146-151 | Exemplary T cell epitope recognized by vaccine-induced T cells (e.g., CD8+ and/or CD4+ T cells) and presented by an MHC class I allele present in at least 50% of subjections in a population |
| 49 | Exemplary amino acid sequence of a prefusion-stabilized SARS-CoV-2 S protein of a BA.1 Omicron variant |
| 50 | Exemplary RNA sequence encoding a prefusion-stabilized SARS-CoV-2 S protein of a BA.1 Omicron variant |
| 51 | Exemplary RNA sequence comprising a sequence encoding a prefusion-stabilized SARS-CoV-2 S protein of a BA.1 Omicron variant, along with 5' UTR, 3'UTR, and polyA sequences |
| 52 | Exemplary amino acid sequence of a prefusion-stabilized SARS-CoV-2 S protein of a BA.1 Omicron variant |
| 53 | Exemplary RNA sequence encoding a prefusion-stabilized SARS-CoV-2 S protein of a BA.1 Omicron variant |
| 54 | Exemplary RNA sequence comprising a sequence encoding a prefusion-stabilized SARS-CoV-2 S protein of a BA.1 Omicron variant, along with 5' UTR, 3'UTR, and polyA sequences |

| | |
|---|---|
| 55 | Exemplary amino acid sequence of a prefusion-stabilized SARS-CoV-2 S protein of a beta variant |
| 56 | Exemplary RNA sequence encoding a prefusion-stabilized SARS-CoV-2 S protein of a beta variant |
| 57 | Exemplary RNA sequence comprising a sequence encoding a prefusion-stabilized SARS-CoV-2 S protein of a beta variant, along with 5' UTR, 3'UTR, and polyA sequences |
| 58 | Exemplary amino acid sequence of a prefusion-stabilized SARS-CoV-2 S protein of an alpha variant |
| 59 | Exemplary RNA sequence encoding a prefusion-stabilized SARS-CoV-2 S protein of an alpha variant |
| 60 | Exemplary RNA sequence comprising a sequence encoding a prefusion-stabilized SARS-CoV-2 S protein of an alpha variant, along with 5' UTR, 3'UTR, and polyA sequences |
| 61 | Exemplary amino acid sequence of a prefusion-stabilized SARS-CoV-2 S protein of a delta variant |
| 62 | Exemplary DNA sequence encoding a prefusion-stabilized SARS-CoV-2 S protein of a delta variant |
| 63 | Exemplary dNA sequence comprising a sequence encoding a prefusion-stabilized SARS-CoV-2 S protein of a delta variant, along with 5' UTR, 3'UTR, and polyA sequences |
| 64 | Exemplary amino acid sequence of a prefusion-stabilized SARS-CoV-2 S protein of an Omicron BA.2 variant |
| 65 | Exemplary RNA sequence encoding a prefusion-stabilized SARS-CoV-2 S protein of an Omicron BA.2 variant |
| 66 | Exemplary DNA sequence encoding a prefusion-stabilized SARS-CoV-2 S protein of an Omicron BA.2 variant |
| 67 | Exemplary RNA sequence comprising a sequence encoding a prefusion-stabilized SARS-CoV-2 S protein of an Omicron BA.2 variant, along with 5' UTR, 3'UTR, and polyA sequences |
| 68 | Exemplary DNA sequence comprising a sequence encoding a prefusion-stabilized SARS-CoV-2 S protein of an Omicron BA.2 variant, along with 5' UTR, 3'UTR, and polyA sequences |
| 69 | Exemplary amino acid sequence of a prefusion-stabilized SARS-CoV-2 S protein of an Omicron BA.4/5 variant |
| 70 | Exemplary RNA sequence encoding a prefusion-stabilized SARS-CoV-2 S protein of an Omicron BA.4/5 variant |
| 71 | Exemplary DNA sequence encoding a prefusion-stabilized SARS-CoV-2 S protein of an Omicron BA.4/5 variant |
| 72 | Exemplary RNA sequence comprising a sequence encoding a prefusion-stabilized SARS-CoV-2 S protein of an Omicron BA.4/5 variant, along with 5' UTR, 3'UTR, and polyA sequences |
| 73 | Exemplary DNA sequence comprising a sequence encoding a prefusion-stabilized SARS-CoV-2 S protein of an Omicron BA.4/5 variant, along with 5' UTR, 3'UTR, and polyA sequences |
| 74 | Exemplary amino acid sequence of a SARS-CoV-2 S protein of a BA.4/5 variant, comprising prefusion-stabilizing mutations |
| 75 | Exemplary RNA sequence encoding a prefusion-stabilized SARS-CoV-2 S protein of an Omicron BA.4/5 variant |
| 76 | Exemplary DNA sequence encoding a prefusion-stabilized SARS-CoV-2 S protein of an Omicron BA.4/5 variant |
| 77 | Exemplary amino acid sequence of a SARS-CoV-2 S protein of a BA.4/5 variant, comprising prefusion-stabilizing mutations |
| 78 | Exemplary RNA sequence encoding a prefusion-stabilized SARS-CoV-2 S protein of an Omicron BA.4/5 variant |
| 79 | Exemplary DNA sequence encoding a prefusion-stabilized SARS-CoV-2 S protein of an Omicron BA.4/5 variant |

| | |
|---|---|
| 80 | Exemplary amino acid sequence of a prefusion-stabilized SARS-CoV-2 S protein of an Omicron BA.2.75 variant |
| 81 | Exemplary RNA sequence encoding a prefusion-stabilized SARS-CoV-2 S protein of an Omicron BA.2.75 variant |
| 82 | Exemplary DNA sequence encoding a prefusion-stabilized SARS-CoV-2 S protein of an Omicron BA.2.75 variant |
| 83 | Exemplary RNA sequence comprising a sequence encoding a prefusion-stabilized SARS-CoV-2 S protein of an Omicron BA.2.75 variant, along with 5' UTR, 3'UTR, and polyA sequences |
| 84 | Exemplary DNA sequence comprising a sequence encoding a prefusion-stabilized SARS-CoV-2 S protein of an Omicron BA.2.75 variant, along with 5' UTR, 3'UTR, and polyA sequences |
| 85 | Exemplary amino acid sequence of a prefusion-stabilized SARS-CoV-2 S protein of an Omicron BA.2.75.2 variant |
| 86 | Exemplary RNA sequence encoding a prefusion-stabilized SARS-CoV-2 S protein of an Omicron BA.2.75.2 variant |
| 87 | Exemplary DNA sequence encoding a prefusion-stabilized SARS-CoV-2 S protein of an Omicron BA.2.75.2 variant |
| 88 | Exemplary RNA sequence comprising a sequence encoding a prefusion-stabilized SARS-CoV-2 S protein of an Omicron BA.2.75.2 variant, along with 5' UTR, 3'UTR, and polyA sequences |
| 89 | Exemplary DNA sequence comprising a sequence encoding a prefusion-stabilized SARS-CoV-2 S protein of an Omicron BA.2.75.2 variant, along with 5' UTR, 3'UTR, and polyA sequences |
| 90 | Exemplary amino acid sequence of a prefusion-stabilized SARS-CoV-2 S protein of an Omicron BA.4.6/BF.7 variant |
| 91 | Exemplary RNA sequence encoding a prefusion-stabilized SARS-CoV-2 S protein of an Omicron BA.4.6/BF.7 variant |
| 92 | Exemplary DNA sequence encoding a prefusion-stabilized SARS-CoV-2 S protein of an Omicron BA.4.6/BF.7 variant |
| 93 | Exemplary RNA sequence comprising a sequence encoding a prefusion-stabilized SARS-CoV-2 S protein of an Omicron BA.4.6/BF.7 variant, along with 5' UTR, 3'UTR, and polyA sequences |
| 94 | Exemplary DNA sequence comprising a sequence encoding a prefusion-stabilized SARS-CoV-2 S protein of an Omicron BA.4.6/BF.7 variant, along with 5' UTR, 3'UTR, and polyA sequences |
| 95 | Exemplary amino acid sequence of a prefusion-stabilized SARS-CoV-2 S protein of an Omicron XBB variant |
| 96 | Exemplary RNA sequence encoding a prefusion-stabilized SARS-CoV-2 S protein of an Omicron XBB variant |
| 97 | Exemplary DNA sequence encoding a prefusion-stabilized SARS-CoV-2 S protein of an Omicron XBB variant |
| 98 | Exemplary RNA sequence comprising a sequence encoding a prefusion-stabilized SARS-CoV-2 S protein of an Omicron XBB variant, along with 5' UTR, 3'UTR, and polyA sequences |
| 99 | Exemplary DNA sequence comprising a sequence encoding a prefusion-stabilized SARS-CoV-2 S protein of an Omicron XBB variant, along with 5' UTR, 3'UTR, and polyA sequences |
| 100 | Exemplary amino acid sequence of a prefusion-stabilized SARS-CoV-2 S protein of an Omicron BQ.1.1 variant |
| 101 | Exemplary RNA sequence encoding a prefusion-stabilized SARS-CoV-2 S protein of an Omicron BQ.1.1 variant |
| 102 | Exemplary DNA sequence encoding a prefusion-stabilized SARS-CoV-2 S protein of an Omicron BQ.1.1 variant |
| 103 | Exemplary RNA sequence comprising a sequence encoding a prefusion-stabilized SARS-CoV-2 S protein of an Omicron BQ.1.1 variant, along with 5' UTR, 3'UTR, and polyA sequences |

| | |
|---|---|
| 104 | Exemplary DNA sequence comprising a sequence encoding a prefusion-stabilized SARS-CoV-2 S protein of an Omicron BQ.1.1 variant, along with 5' UTR, 3'UTR, and polyA sequences |
| 105 | Exemplary HSV-1 gD signal peptide |
| 106 | Exemplary HSV-2 gD signal peptide |
| 107 | Exemplary HSV-2 signal peptide |
| 108 | Exemplary SARS-CoV-2 Spike signal peptide |
| 109 | Exemplary human Ig heavy chain signal peptide (huSec) |
| 110 | Exemplary HuIgGk signal peptide |
| 111 | Exemplary IgE heavy chain epsilon-1signal peptide |
| 112 | Exemplary Japanese encephalitis PRM signal sequence |
| 113 | Exemplary VSVg protein signal sequence |
| 114-119 | Exemplary signal peptides |
| 120 | Exemplary nucleotide sequence encoding an HSV-1 gD signal peptide |
| 121 | Exemplary nucleotide sequence encoding an HSV-1 gD signal peptide |
| 122 | Exemplary nucleotide sequence encoding a SARS-CoV-2 Spike signal peptide |
| 123 | Exemplary nucleotide sequence encoding a human Ig heavy chain signal peptide (huSec) |
| 124 | Exemplary epitope In a Wuhan SARS-CoV-2 S protein |
| 125 | Exemplary epitope in a SARS-CoV-2 S protein of a delta variant comprising an L452R mutation |
| 126 | Exemplary epitope in SARS-CoV-2 Wuhan strain RBD |
| 127 | Exemplary epitope in an RBD of a delta variant of SARS-CoV-2 |
| 128 | Exemplary epitope in an RBD of Omicron BA.1 and BA.4/5 variant of SARS-CoV-2 |
| 129 | Exemplary amino acid sequence of an RBD of a SARS-CoV-2 alpha variant |
| 130 | Exemplary amino acid sequence of an RBD of a SARS-CoV-2 beta variant |
| 131 | Exemplary amino acid sequence of an RBD of a SARS-CoV-2 delta variant |
| 132 | Exemplary amino acid sequence of an RBD of a SARS-CoV-2 Omicron BA.1 variant |
| 133 | Exemplary amino acid sequence of an RBD of a SARS-CoV-2 Omicron BA.4/5 variant |
| 134 | Exemplary GS linker |
| 135 | Exemplary GS linker |
| 136 | Furin cleavage site in SARS-CoV-2 S protein |
| 137 | Exemplary nucleotide sequence encodign a furin cleavage site in SARS-CoV-2 S protein |
| 152 | Exemplary DNA sequence encoding a prefusion-stabilized SARS-CoV-2 S protein of an Beta variant |
| 153 | Exemplary DNA sequence comprising a sequence encoding a prefusion-stabilized SARS-CoV-2 S protein of a Beta variant, along with 5' UTR, 3'UTR, and polyA sequences |
| 154 | Exemplary DNA sequence encoding a prefusion-stabilized SARS-CoV-2 S protein of an Alpha variant |
| 155 | Exemplary DNA sequence comprising a sequence encoding a prefusion-stabilized SARS-CoV-2 S protein of an Alpha variant, along with 5' UTR, 3'UTR, and polyA sequences |
| 156 | Exemplary RNA sequence encoding a prefusion-stabilized SARS-CoV-2 S protein of a Delta variant |
| 157 | Exemplary RNA sequence comprising a sequence encoding a prefusion-stabilized SARS-CoV-2 S protein of a Delta variant, along with 5' UTR, 3'UTR, and polyA sequences |
| 158 | Exemplary amino acid sequence of a prefusion-stabilized SARS-CoV-2 S protein of an Omicron XBB.1.5 variant |
| 159 | Exemplary RNA sequence encoding a prefusion-stabilized SARS-CoV-2 S protein of an Omicron XBB.1.5 variant |

| | |
|---|---|
| 160 | Exemplary DNA sequence encoding a prefusion-stabilized SARS-CoV-2 S protein of an Omicron XBB.1.5 variant |
| 161 | Exemplary RNA sequence comprising a sequence encoding a prefusion-stabilized SARS-CoV-2 S protein of an Omicron XBB.1.5 variant, along with 5' UTR, 3'UTR, and polyA sequences |
| 162 | Exemplary DNA sequence comprising a sequence encoding a prefusion-stabilized SARS-CoV-2 S protein of an Omicron XBB.1.5 variant, along with 5' UTR, 3'UTR, and polyA sequences |
| 163 | Exemplary amino acid sequence of a prefusion-stabilized SARS-CoV-2 S protein of an Omicron XBB.1.16 variant |
| 164 | Exemplary RNA sequence encoding a prefusion-stabilized SARS-CoV-2 S protein of an Omicron XBB.1.16 variant |
| 165 | Exemplary DNA sequence encoding a prefusion-stabilized SARS-CoV-2 S protein of an Omicron XBB.1.16 variant |
| 166 | Exemplary RNA sequence comprising a sequence encoding a prefusion-stabilized SARS-CoV-2 S protein of an Omicron XBB.1.16 variant, along with 5' UTR, 3'UTR, and polyA sequences |
| 167 | Exemplary DNA sequence comprising a sequence encoding a prefusion-stabilized SARS-CoV-2 S protein of an Omicron XBB.1.16 variant, along with 5' UTR, 3'UTR, and polyA sequences |
| 168 | Exemplary amino acid sequence of a prefusion-stabilized SARS-CoV-2 S protein of an Omicron XBB.2.3 variant |
| 169 | Exemplary RNA sequence encoding a prefusion-stabilized SARS-CoV-2 S protein of an Omicron XBB.2.3 variant |
| 170 | Exemplary DNA sequence encoding a prefusion-stabilized SARS-CoV-2 S protein of an Omicron XBB.2.3 variant |
| 171 | Exemplary RNA sequence comprising a sequence encoding a prefusion-stabilized SARS-CoV-2 S protein of an Omicron XBB.2.3 variant, along with 5' UTR, 3'UTR, and polyA sequences |
| 172 | Exemplary DNA sequence comprising a sequence encoding a prefusion-stabilized SARS-CoV-2 S protein of an Omicron XBB.2.3 variant, along with 5' UTR, 3'UTR, and polyA sequences |
| 173 | Exemplary amino acid sequence of a prefusion-stabilized SARS-CoV-2 S protein of an Omicron XBB.2.3.2 variant |
| 174 | Exemplary RNA sequence encoding a prefusion-stabilized SARS-CoV-2 S protein of an Omicron XBB.2.3.2 variant |
| 175 | Exemplary DNA sequence encoding a prefusion-stabilized SARS-CoV-2 S protein of an Omicron XBB.2.3.2 variant |
| 176 | Exemplary RNA sequence comprising a sequence encoding a prefusion-stabilized SARS-CoV-2 S protein of an Omicron XBB.2.3.2 variant, along with 5' UTR, 3'UTR, and polyA sequences |
| 177 | Exemplary DNA sequence comprising a sequence encoding a prefusion-stabilized SARS-CoV-2 S protein of an Omicron XBB.2.3.2 variant, along with 5' UTR, 3'UTR, and polyA sequences |

## Claims

1. A composition comprising an RNA molecule having:
(a) a nucleotide sequence that is at least 99% identical to SEQ ID NO: 161;
(b) a nucleotide sequence that is at least 95% identical to SEQ ID NO: 161, and which encodes a SARS-CoV-2 S protein comprising the following mutations relative to SEQ ID NO: 1: T19I, Δ24-26, A27S, V83A, G142D, Δ145, H146Q, Q183E, V213E, G252V, G339H, R346T, L368I, S371F, S373P, S375F, T376A, D405N, R408S, K417N, N440K, V445P, G446S, N460K, S477N, T478K, E484A, F486P, F490S, Q498R, N501Y, Y505H, D614G, H655Y, N679K, P681H, N764K, D796Y, Q954H, N969K, K986P, and V987P; or
(c) a nucleotide sequence as set forth in SEQ ID NO: 161; and
wherein the RNA molecule comprises:
(i) a 5' cap; and
(ii) a modified uridine in place of each uridine.

2. The composition of claim 1, wherein the modified uridine is N1-methyl-pseudouridine.

3. The composition of claim 1 or claim 2, wherein the 5' cap comprises m₂^{7,3'-O}Gppp(m₁^{2'-O})ApG.

4. The composition of any one of claims 1 to 3, wherein the RNA molecule is encapsulated in a lipid nanoparticle (LNP), preferably wherein the LNP comprises molar ratios of 20-60% ionizable cationic lipid, 5-25% neutral lipid, 25-55% sterol, and 0.5-15% PEG-modified lipid.

5. The composition of any one of claims 1 to 4, wherein the composition comprises one or more additional RNA molecules, each having a nucleotide sequence encoding an S protein of a SARS-CoV-2 strain or variant that is not XBB.1.5, preferably wherein the one or more additional RNA molecules comprise a sequence that is at least 95% identical to that set forth in SEQ ID NO: 20, 72, or 103.

6. The composition of any one of claims 1 to 5, wherein the RNA molecule comprises:
(i) N1-methyl-pseudouridine in place of each uridine; and
(ii) a 5' cap that comprises m₂^{7,3'-O}Gppp(m₁^{2'-O})ApG;
wherein the RNA molecule is encapsulated in a lipid nanoparticle (LNP); and
wherein the LNP comprises molar ratios of 20-60% ionizable cationic lipid, 5-25% neutral lipid, 25-55% sterol, and 0.5-15% PEG-modified lipid.

7. The composition of any one of claims 1 to 6, comprising about 10 mM Tris buffer and about 10% sucrose.

8. The composition of any one of claims 1 to 7, wherein the composition comprises at least one unit dose of LNP-encapsulated RNA molecules, optionally wherein the unit dose comprises the RNA molecule in an amount of about 30 µg, or wherein the unit dose comprises the RNA molecule in an amount of about 10 µg, or wherein the unit dose comprises the RNA molecule in an amount of about 3 µg.

9. The composition of any one of claims 1 to 8, wherein the composition is formulated as a multi-dose formulation in a vial.

10. A composition of any one of claims 1 to 9 for use in a method of inducing an immune response against coronavirus in a subject, said method comprising administering to a subject the composition.

11. The composition for use of claim 10, wherein:
the subject is 12 years or older, and the composition comprises 30 µg of the RNA molecule, or
the subject is 5 years to less than 12 years old, and the composition comprises 10 µg of the RNA molecule,
or
the subject is 6 months to less than 5 years old, and the composition comprises 3 µg of the RNA molecule.

12. The composition for use of claim 10 or claim 11, wherein the composition is administered in a volume of about 200 µL to 300 µL.

13. The composition for use of any one of claims 10 to 12, wherein the subject was previously administered one or more doses of a SARS-CoV-2 vaccine, preferably wherein the subject was previously administered a complete dosing regimen of a SARS-CoV-2 vaccine.

14. The composition for use of any one of claims 10 to 13, wherein the subject was previously administered a first dose and a second dose of BNT162b2, wherein the first dose and the second dose were administered about 21 days apart, and/or wherein the subject was previously administered as a booster dose a bivalent vaccine that delivers (i) a SARS-CoV-2 S protein of an Omicron BA.4/5 variant and (ii) a SARS-CoV-2 S protein of a Wuhan strain.

15. The composition for use of any one of claims 10 to 14, wherein said method further comprises administering one or more vaccines against a non-SARS-CoV-2 disease, preferably wherein the one or more vaccines comprises an RSV vaccine, an influenza vaccine, or a combination thereof.
